# EUROPEAN PATENT APPLICATION

(11) **EP 2 438 908 A1**
(43) Date of publication of application: **11.04.2012**
(21) Application number: 10382265.6
(22) Date of filing: 11.10.2010
(51) Int. Cl.: A61K 8/81, A61Q 17/04, A61Q 19/00

(54) **Anchoring compositions for topical applications**

(71) Applicant: Vectum Pharma, S.L., 08008 Barcelona (ES)
(72) Inventor: Ramos Pérez, Víctor, 08008, Barcelona (ES); Borrós Gómez, Salvador, 08173, Sant Cugat del Vallés (ES); Fisher, Kerry David, Eynsham, Oxfordshire OX294ET (GB)
(74) Representative: Elzaburu Marquez, Alberto

(57) **Abstract**

Disclosed is an amphiphilic copolymer that can be used in film-forming compositions for dermal applications. The amphiphilic copolymer is characterized by having a defined structure with a hydrophilic polymer backbone and pendant hydrophobic groups. When applied to the skin, the copolymer rearranges in contact with the lipid-rich domains of the outermost layer of the skin, resulting in stable film formation. The amphiphilic copolymer contains active ingredients covalently attached, such as sunscreens, humectants or colourings, resulting in skin immobilization of the active ingredient. The amphiphilic copolymer can also contain other active agents, such as fragrances, cosmeceuticals or dermatologically active drugs, covalently attached via a chemical bond that can be degraded under the physiological conditions found on the skin, resulting in sustained release of the immobilized active ingredient.

## Description

### Field of the invention

The invention relates generally to water-soluble polymer-active ingredient conjugates, to a method to deliver an active ingredient to the skin of an animal comprising applying said water-soluble conjugates over the skin, and to the use of said water-soluble polymer-active ingredient conjugates to deliver an active ingredient to the skin of an animal, such as a human.

### Background

The skin is by far the largest organ of the human body and its main function is to protect the body against external mechanical, chemical and microbiological challenges. The skin has been classically divided into three main layers: epidermis (upper layer, non-vascularized), dermis (middle layer, vascularized) and hypodermis (bottom or innermost layer). In addition, these regions are further divided in other more specific and thinner layers, conferring the skin its characteristic stratified structure.

The stratum corneum is the outermost layer of the skin (epidermis) and has an approximate thickness of 10 - 20 µm. This layer is mainly composed of corneocytes and an intercellular lipid matrix organized as multilamellar bilayers. Corneocytes are non-viable terminally differentiated keratinocytes that have lost their cellular organelles. The intercellular lipid matrix consists mainly of ceramides, cholesterol and fatty acids (Elias⁹, Lampe¹⁶). The stratum corneum has been described as a "brick and mortar" organization, where corneocytes represent the bricks, and the intercellular lipid matrix organized as multilamellar bilayers resembles the connecting mortar (Schmidt-Werdner²⁵). The external side of the stratum corneum is covered by a slightly acidic aqueous film with antimicrobial properties, known as the acid mantle (Rippke²⁴).

The skin functions as an important barrier against the penetration of foreign substances and microorganisms into the human body. For this reason, healthy skin not only provides an attractive appearance, but also maintains important barrier functions. Many efforts have been made to obtain therapeutic products to beautify, protect and treat the skin via topical administration. Topical dermal preparations are designed to localize their therapeutic effect at the application site by delivering their active ingredients on the surface or into the underlying layers of skin. Topical preparations include a wide range of formulation systems, including foams, solutions, ointments, gels, powders and even adhesive patches. The physico-chemical properties of active ingredients and vehicles are considered to be the main features responsible for the distribution of the active ingredients in the different skin layers. Topical administration of active ingredients is still a challenge in pharmaceutics and cosmetics due to the difficulty in controlling and determining the exact amount of therapeutic agent that reaches the different skin layers (Bronaugh⁵, Surber²⁸). For this reason, delivery systems that enable control of the pharmacological profile of topically administered ingredients are extensively investigated (Nino¹⁹, Langer³⁴).

Topical therapy of skin diseases allows high drug levels at the site of disease and reduces systemic side effects. However, pharmaceutical formulations for the treatment of skin diseases are generally characterized by poor drug uptake and high drug-absorption variability among individuals. In addition, the use of topical drugs is sometimes associated with unwanted local side effects, e.g. skin atrophy following extended administration of corticoids (Ermis¹⁰), or irritation in anti-acne treatments (Thielitz²⁹). To improve topic administration of drugs, efficient and well-tolerated delivery carrier systems are continuously being developed. Pharmaceutical formulations with the ability to deliver therapeutic drug levels of drug over defined periods of time provide significant advantages over immediate release formulations. Sustained release formulations are administered in much fewer daily doses and generally achieve improved therapeutic effect and efficiency. In addition, topical preparations that enable drug targeting to specific skin layers may improve the efficacy of active ingredients and reduce local unwanted effects.

In the cosmetic and cosmeceutical fields, there is an increasing awareness of developing effective products with proven clinical efficacy. The delivery of active ingredients is sometimes hampered due to some problems, such as skin irritation, low skin permeability and chemical instability of compounds sensitive to light, hydrolysis or oxidation. The need for dermal delivery systems that not only provide superior release and distribution profiles of the active agents within or on the skin, but also simplified formulations with improved organoleptic properties has long been a concern in areas such as cosmetics, cosmeceuticals and personal care.

In order to overcome delivery limitations associated with topical administration of active ingredients, new topical delivery systems are continuously being developed. Recent progress in nanotechnology may allow developing alternative delivery systems with improved skin penetration and even targeting properties. This is applicable to liposomes (Honeywell-Nguyen¹³), nanoemulsions, solid-lipid nanoparticles (Santos-Maia²³) and polymeric nanoparticles (Alvarez-Roman³), which can enhance drug uptake to the epidermis. The use of these systems provides advantageous features for topic application of cosmetic ingredients and drugs, such as controlled or modified release, improved stability, skin targeting, improved skin compatibility, increase of skin penetration and/or reduction of systemic uptake.

Film-forming polymeric preparations are an alternative to conventional dosage forms used for skin drug delivery, such as ointments, gels or patches. In this approach, a polymeric solution is applied to the skin as a solution (or in some cases as low viscous gels or creams) and an almost invisible film is formed after solvent evaporation. Typically, film-forming preparations have been used as tissue glue (Singer²⁶), disinfectants (Jeng¹⁴) and liquid bandages for the care of minor cuts and abrasions (Eaglstein⁸). In contrast, only few applications have described the use of film-forming preparations for the delivery of active ingredients to the skin. In such applications, the preparations form thin polymeric films after solvent evaporation, from which the drug is released to the skin. Examples of such film-forming preparations for drug delivery have been investigated as an alternative delivery system for steroidal hormone treatments (Misra¹⁸). Ideally, such preparations dry quickly on the skin, forming homogeneous, stable and almost invisible films. The formed films are required to show extended residence on skin, in order to provide a continuous active ingredient supply over a prolonged period of time. This means that films are required to be non-sticky, non-transferable, ideally water- and sweat-resistant, and have appropriate mechanical properties, such as flexibility and abrasion resistance. In addition, films should be compatible with skin chemistry and do not interfere with normal skin physiological processes, such as transpiration, desquamation or pH regulation.

Film-forming formulations are an attractive alternative to currently available dosage formulations with unmet needs. Due to their unique features, film-forming formulations are halfway between dermal patches and semi-solid formulations. They can provide many advantages over patches, such as greater cosmetical attractiveness and improved dosing flexibility, when applied with a metered-dose applicator. When compared to semi-solid preparations, film-forming formulations may allow prolonged delivery of active ingredients and improved organoleptic properties, such as extended wear and reduced staining or stickiness. Film-forming compositions differ from semi-solid formulations containing polymeric species in that the latter do not form homogeneous films, when applied on the surface of the skin. In semi-solid formulations, polymers are generally used as gelling agents to increase the viscosity of the dermal preparation.

The patent literature also discloses widely different compositions claiming to be able to deliver different active agents to keratinous substrates:
In certain cases, the claimed compounds are intended to deliver an agent of interest to cells, such as in W02009/011702, where drug carriers are used to deliver certain drugs to cancer cells, or in W02009/064696 where the compounds are said to deliver certain agents such as drugs, imaging agents or contrast materials to cells. However, these compounds are not necessarily able to bond or attach onto the skin and anchor to the stratum corneum to hold the active ingredient in place, thus allowing its controlled release. Similar teachings can be found e.g. in WO 01/037665 and WO98/19710.

In other cases, dermal barrier compositions are disclosed, which can contain active agents such as sunscreens, insect repellent or fungicides. Examples of these documents are US 6,582,682, W02005/078956 and W02010/091274. These compositions however usually comprise a combination of compounds having different properties. These combinations are however not really skin-anchoring compositions and therefore, following the application of the composition, either leave fat deposits over the skin or require rinsing of the non-active carrier ingredients. Moreover, these compositions do not really attach to the skin, but instead their skin-bonding properties arise from their insolubility in water, which affords them a high residence time on the skin. This is the case e.g. of sunscreen compositions.

A third group of references is directed to compositions claiming to deliver agents of interest onto a keratinous substrate such as the stratum corneum, such as e.g. in WO 2009/130144 issued to BASF SE, where amphiphilic block polymers were used to deliver personal care agents to the skin; US 5,906, 822 and US 5,911,980, both issued to Samour, where cationic film-forming polymers were used to deliver beneficial compounds to the skin or hair; WO 2007/016146, where polymeric film-forming polymers were used to retard the rate at which drugs such as hormones penetrate into and through the skin, or US 6,436,412, issued to Quinn, where hybrid polymeric materials containing functional groups capable of forming hydrogen bonds were disclosed for coating keratinous materials. However, the compounds disclosed in these documents do not anchor to the skin making use of a multiplicity of anchoring moieties, or in other words they are not multivalent, so that they cannot take benefit of the improved stability of the skin-bonding compositions according to the present invention.

Finally, in certain cases the active ingredient optionally incorporates a linker cleavable by an exogenous agent, so that the active ingredient can be released by providing said exogenous agent in a controlled manner, such as in EP 0 245 858, WO98/50040 or WO2009/038776.

In all these cases, the disclosed compounds or compositions suffer of one or more of the following drawbacks:
1) Since the compounds or compositions do not really attach onto the skin, their resistance to wear or water, or in other words the residence time of the active ingredient over the skin, is low and therefore these compositions do not allow an extended and controlled release of the active ingredient over the skin;
2) The compositions contain carriers or inactive ingredients that leave unpleasant deposits on the skin surface and/or require rinsing of the non-active ingredients to operate properly;
3) The compositions are not film-like and therefore their presence cannot go unnoticed, which in certain circumstances can be disadvantageous.

### Summary of the invention

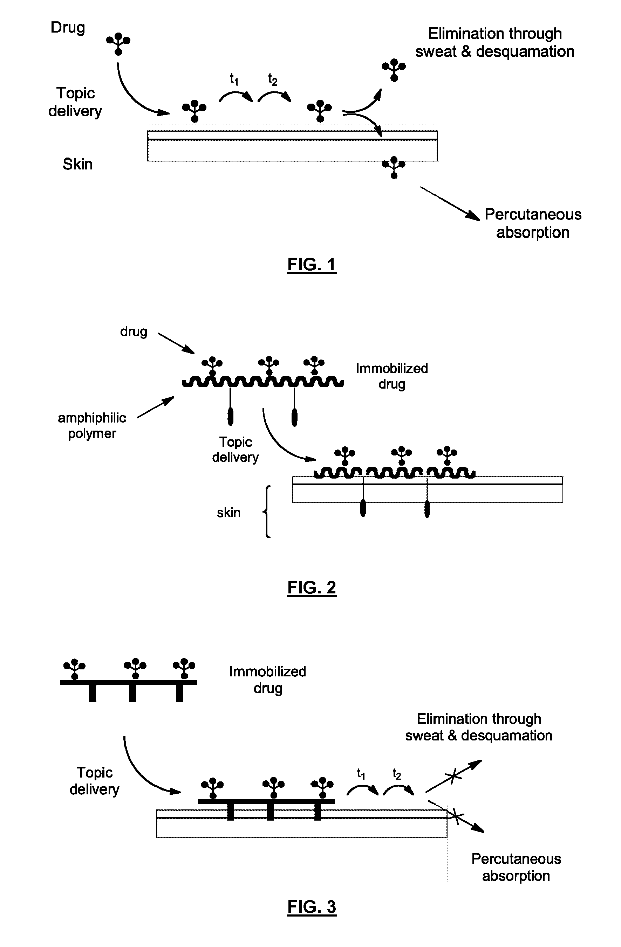

Therefore the problem to be solved by the present invention is to provide skin-bonding or skin-anchoring compositions, which are able to deliver or hold in place active ingredients on the skin surface for prolonged periods of time and in a controlled manner, overcoming the disadvantages of the prior art (figure 1).

The solution is based on that the present inventors have identified that by providing amphiphilic copolymers based on monomers derived from acrylic acid, methacrylic acid or any water-soluble vinyl monomer (e.g. N-vinylpyrrolidone), such as a poly(meth)acrylamide or particularly poly[N-(2-hydroxypropyl)-methacrylamide, which amphiphilic copolymers have a number-average molecular weight of at least 50,000 g/mol and comprise at least 1 mol% of monomers having at least one pendant hydrophobic group able to bind to the skin of an animal by hydrophobic interactions, compositions having extended skin-anchoring properties are obtained, therefore allowing an extended and controlled delivery of active principles to the skin, particularly for cosmetic and dermatological applications. The active ingredients are covalently linked to the backbone of the copolymer chain, preferably via linkers cleavable with an exogenous cleaving agent such as e.g. enzymes, a low pH or a reducing medium, so that cleavage may be triggered by providing said cleaving agent in a controlled manner.

Accordingly, a first aspect of the invention is directed to a water-soluble polymer-active ingredient conjugate comprising:
- at least one hydrophilic copolymer chain comprising monomers derived from acrylic acid, methacrylic acid or any water-soluble vinyl monomer in any combination thereof; and
- at least one active ingredient linked to the copolymer chain through a linker group having at least one covalent bond;
characterized in that the copolymer chain has a number-average molecular weight of at least 50,000 g/mol and comprises at least 1 mol% of monomers having at least one pendant hydrophobic group able to bind the copolymer chain to the skin of an animal by hydrophobic interactions.

A second aspect of the invention relates to a method to deliver an active ingredient over the skin of an animal, comprising the steps of applying said water-soluble conjugates over the skin and allowing the solvent to evaporate, thereby forming a film over the skin having the active ingredient covalently attached thereto.

A third aspect of the invention relates to the use of said polymer-active ingredient conjugates to deliver an active ingredient to the skin of an animal.

The compositions of the invention have additional advantages. For instance, since they are mostly hydrophilic, they are water-soluble. Therefore they can be formulated as water-borned compositions in such a manner that their organoleptic characteristics are more appealing for the user and deposition of oily residues on the skin is avoided.

The compounds of the invention can be generally described as amphiphilic random copolymers, characterized by having pendant hydrophobic groups attached to a hydrophilic polymer backbone. Amphiphilic copolymers are able to associate with the outermost layer of the skin (stratum corneum), resulting in stable and uniform skin coating. Polymer association with skin is mediated by hydrophobic interactions driving the pendant groups to insert into the hydrophobic region of the stratum corneum (intercellular lipid matrix and lipids attached to the cornified envelope of corneocytes), while the hydrophilic polymer backbone remains localized on the surface (figure 2).

As a result of the provision of a multiplicity of pendant hydrophobic groups per polymer chain, the copolymers are able to bind to the skin of an animal via hydrophobic interactions. When inserted into the stratum corneum each hydrophobic group has an additive effect, resulting in a co-operative effect that contributes to the stability of anchoring to the skin of the whole polymer. The higher stability of the compounds of the invention is thus explained by the greater length of its copolymer chains, which corresponds to a higher number of hydrophobic groups per polymer chain. Consequently, films prepared with the above-cited amphiphilic copolymers are stable against wash off in water. Conjugation of active ingredients to amphiphilic polymers results in stable immobilization of active ingredients on the stratum corneum. In this scenario, polymer-coated skin works as a reservoir of active ingredient. However, depending on the purpose of the active ingredient, the chemical linkage between copolymer and active ingredient can be designed as stable or degradable, resulting in either extended residence on the top layer of the skin or release of the active ingredient over a period of time respectively.

### Brief description of the figures

**Figure 1****.** Schematic representation of topic delivery.
**Figure 2****.** Schematic representation of immobilization of active ingredients using drug-conjugated amphiphilic polymers.
**Figure 3****.** Schematic representation of drug immobilization using drug-conjugated amphiphilic polymers.
**Figure 4****.** Schematic representation of extended release from active ingredient copolymer conjugate immobilized on the skin surface.
**Figure 5****.** Schematic representation of a hydrophilic copolymer with pendant hydrophobic groups.
**Figure 6****.** Schematic representation of the synthesis of the compounds of the invention by direct copolymerisation. Amphiphilic polymers bearing humectant moieties were prepared by on pot copolymerisation of hydrophilic (HPMA), hydrophobic (methacryloylated cholesterol moieties) and drug monomers (beta-hydroxyethylurea).
**Figure 7****.** Example of synthesis of polymer-drug conjugates by modification of preformed amphiphilic polymers. Amphiphilic polymers bearing UV filter moieties were prepared via a two-step procedure. First, reactive amphiphilic polymers were obtained by copolymerisation of hydrophilic (HPMA), hydrophobic (methacryloylated cholesterol moieties) and reactive monomers (TT groups). Compounds according to the invention were obtained by subsequent reaction of reactive polymers and amine-containing drugs (amine-derivative of transcinnamate).
**Figure 8****.** Schematic representation of a hydrophilic copolymer with pendant hydrophobic groups.
**Figure 9****.** Schematic representation of an amphiphilic copolymer bearing reactive groups.
**Figure 10****.** Cross section of full porcine skin coated with fluorescently labelled of pHPMA-chol(2.5%)-TT(10%) copolymer, viewed under normal (left) and fluorescent light (right).
**Figure 11****.** Stability of coating layers of pork skin coated with control (left) or amphiphilic copolymer (right) after 5 (b), 30 (c) and 60 min (d) incubation in phosphate buffered saline vs. time zero (a).
**Figure 12****.** Removal of polymer coating from the surface of pork skin. The coated skin surface was rubbed with a sponge that had been previously soaked in a soapy solution.
**Figure 13****.** Water resistance of films prepared on the surface of human skin.
Dye-labelled amphiphilic copolymers (pHPMA-chol(1, 2.5 or 5%)-dye, 2 µL at 5 mg/mL in H₂O/EtOH, 1:1) or control co-polymer without pendant hydrophobic groups (pHPMA-dye, 2 µL at 5 mg/mL in H₂O/EtOH, 1:1) were applied on the skin surface of a human forearm. Stripping and spectrophotometric quantification were used to quantify the amount of copolymer before and after 45 min incubation in saline solution.

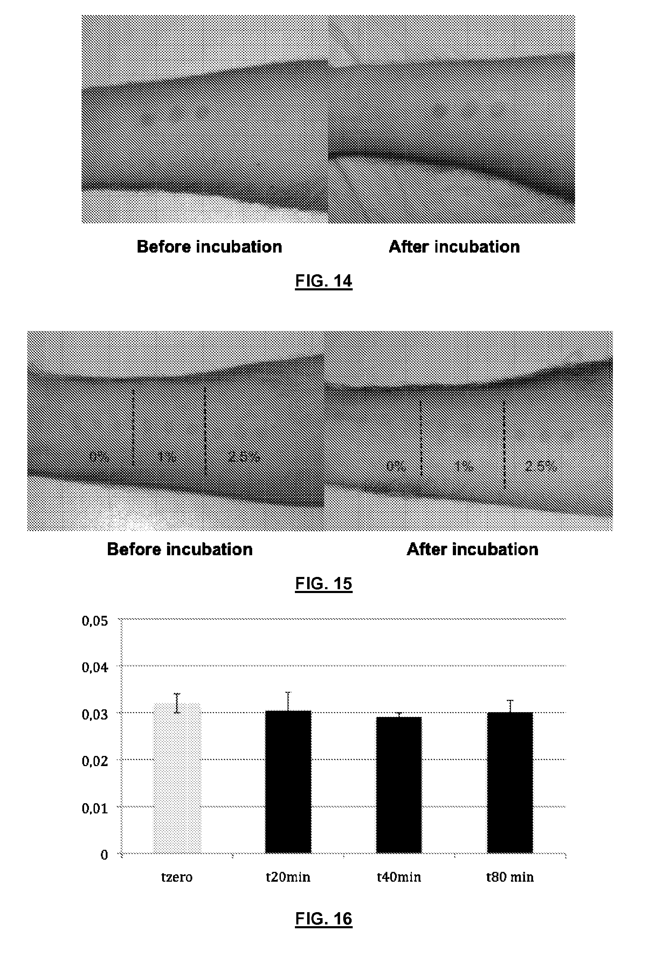

**Figure 14****.** Water resistance of pHPMA-chol(5%)-dye deposited on the skin of a human forearm before (left) and after (right) incubation.
**Figure 15****.** Water resistance of pHPMA-chol(0, 1 and 2.5%)-dye deposited on the skin of a human forearm before (left) and after (right) incubation.
**Figure 16****.** Water resistance of films prepared with pHPMA-chol(5%)-dye on the surface of human skin. Dye-labelled amphiphilic pHPMA-chol(5%)-dye copolymer (2 µL at 5 mg/mL in H₂O/EtOH, 1:1) was applied on the skin surface of a human forearm. Stripping and spectrophotometric quantification were used to quantify the amount of copolymer before and after 1, 2 and 4 incubation cycles in saline solution.

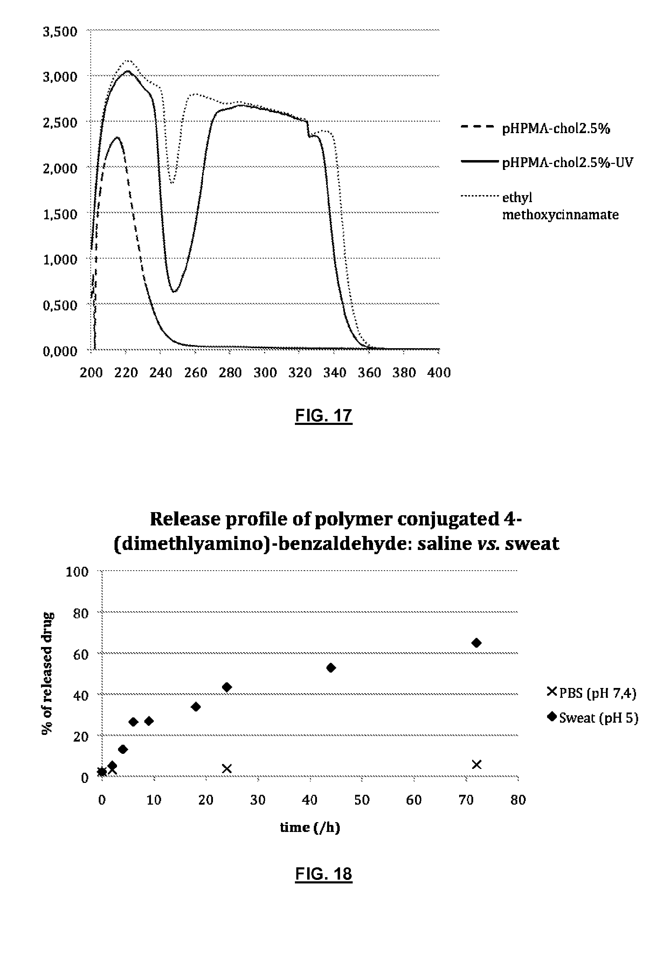

**Figure 17****.** UV-Vis spectra pHPMA-chol(2.5%) (- - -, 1 mg/mL), ethyl-p-methoxycinnamate (...., 0,2 mg/mL) and pHPMA-chol(2.5%)-UV (—,1 mg/mL) dissolved in HPLC grade methanol.
**Figure 18****.** Release profile of pHPMA-chol(2.5%)-DMAB in phosphate buffered saline (× 1 mg/mL, pH 7.4) and artificial sweat (◆, 1 mg/mL, pH 5.0).

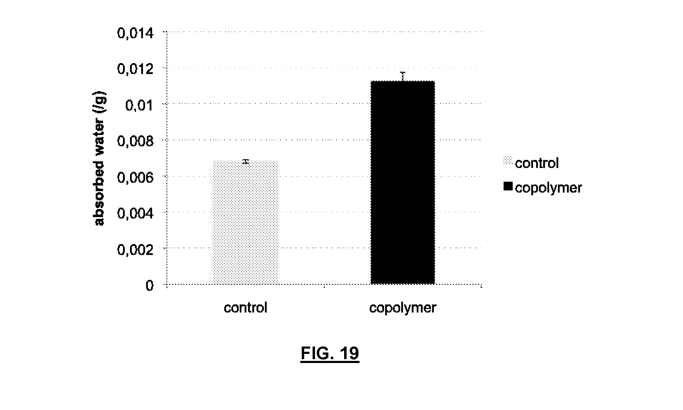

**Figure 19****.** Water absorption of pHPMA-chol(2.5%)-AP vs. control.

### Definitions

Prior to a discussion of the detailed embodiments of the invention is provided a definition of specific terms related to the main aspects of the invention:
"Amphiphilic copolymers" are meant to be polymers characterized by having pendant hydrophobic groups attached to a hydrophilic polymer backbone. When those pendant hydrophobic groups are able to bind to the *stratum corneum* of the skin of an animal, they are also called "anchoring moieties".
The "active ingredient" or "active moiety" is an ingredient of interest to be delivered to the skin, which is covalently bonded to the hydrophilic polymer preferably via a cleavable linker.
"Skin-bonding" or "skin-anchoring" compositions are meant to be compositions that, when applied to the skin, bond to the skin and hold the active ingredient in place on the skin. Depending on the purpose of the active ingredient, the chemical linkage between copolymer and active ingredient can be designed as stable or cleavable, resulting in either extended residence on the top layer of the skin or the controlled release of the active ingredient over a period of time, respectively.
"Reversible" or "cleavable" link refers to spontaneous chemical transformation of a specific bond that connects the active ingredient to the copolymer through a mechanism found on the skin (or that can be accessed from the skin), within a finite and predictable period.
An "anchoring moiety" is a moiety able to bind to the stratum corneum of the skin of an animal, particularly by hydrophobic interactions.
A "multivalent" or "polyvalent" polymer is a polymer that contains a plurality of anchoring moieties.
The "skin of an animal" is meant to be the skin of any animal, particularly the human being.

Embodiments of the invention are described below, by way of examples only.

### Detailed description of the invention

The invention relates to skin-bonding compositions for topical applications, which are particularly suitable for dermatology, cosmetics and personal care products, in order to immobilize active agents on the outermost layer of the skin. The proposed copolymer is held to the outermost layer of the skin through specific hydrophobic interactions between the hydrophobic pendant groups of the copolymer and the intercellular lipid matrix of the stratum corneum. This means that when the copolymer is applied to the skin, its structure undergoes conformational changes. As a result of conformational changes, copolymer chains reorganize and the hydrophobic pendant groups insert into the intercellular lipid matrix. Consequently, each copolymer chain becomes bound to the skin by repeating hydrophobic interactions. The three-dimensional nature of the skin to which the copolymer is applied drives the rearrangement of the copolymer chains along the surface. This rearrangement facilitates the formation of a defined, almost invisible and highly stable film.

The preferred embodiments of this invention include methods of preparing films on the surface of the skin, as previously described, having space suitable for holding desired active ingredients, such as humectants, sunscreens, fragrances, disinfectants, insect repellents, pigments or colourings, cosmetically and dermatologically active ingredients or drugs.

In a preferred embodiment, the skin-anchoring composition is designed to carry and hold an active ingredient in proximity to the skin surface, preventing percutaneous absorption and resulting in extended wear. In such applications, the active ingredient is covalently attached to the amphiphilic copolymer. The high molecular weight of the active ingredient-copolymer conjugate inhibits the absorption of active ingredients towards deeper skin layers or even systemic circulation. In addition, the formation of sweat- and water-resistant active ingredient-copolymer conjugate films prevents washing off of the active ingredient from the skin surface (figure 3). These features are especially interesting for preparing formulations containing active ingredients, whose therapeutic action is mainly targeted at the outermost skin layer, stratum corneum. In such applications, reduced percutaneous absorption is highly desirable, in order to prevent possible adverse effects, such as irritation events or even body accumulation.

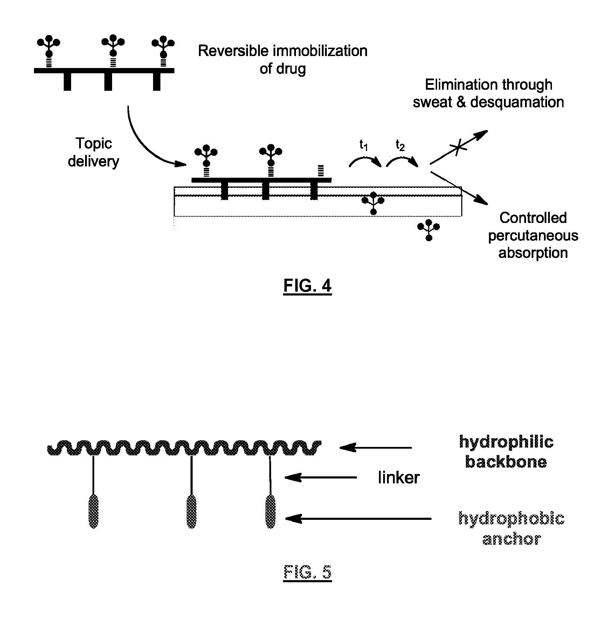

In another preferred embodiment, the film-anchoring composition is designed to work as a reservoir of active ingredients, in order to prepare formulations with extended release profiles. In such applications, the active ingredient is covalently attached to the amphiphilic copolymer via a reversible bond that can be cleaved by a trigger mechanism found on the skin surface. As previously described, copolymer conjugation of active ingredients prevents percutaneous absorption and extends skin residency. These features allow holding an active ingredient on the skin surface as an inactive form, which can be activated upon cleavage of the bond that connects the active ingredient and the copolymer. Following release, free active ingredient can be either percutaneously absorbed or remain on the skin surface, depending on the physico-chemical properties and the target site of the active principle (figure 4). In this scenario, films prepared from amphiphilic copolymers work as drug reservoirs of active ingredients for extended release, similar to delivery systems based on dermal patches, but with improved visual attractiveness.

### Amphiphilic Copolymer Composition

As previously described, the amphiphilic copolymer used in the present invention may include hydrophilic and hydrophobic monomers, which are copolymerized together. In a preferred embodiment of the invention, the resulting amphiphilic copolymer is a linear chain with randomly alternating monomers. In an even more preferred embodiment, the copolymer is composed of a hydrophilic linear chain, from where pendant hydrophobic groups are attached via a spacer or linker. The proposed copolymer has the general structure shown in figure 5.

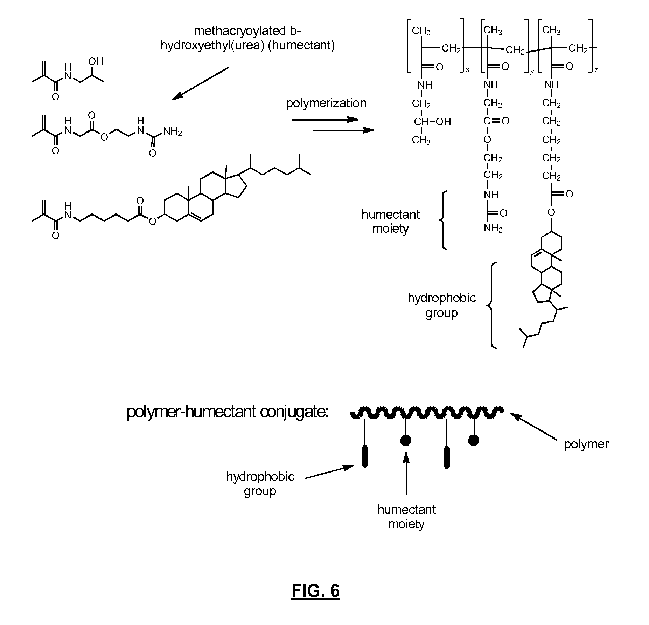

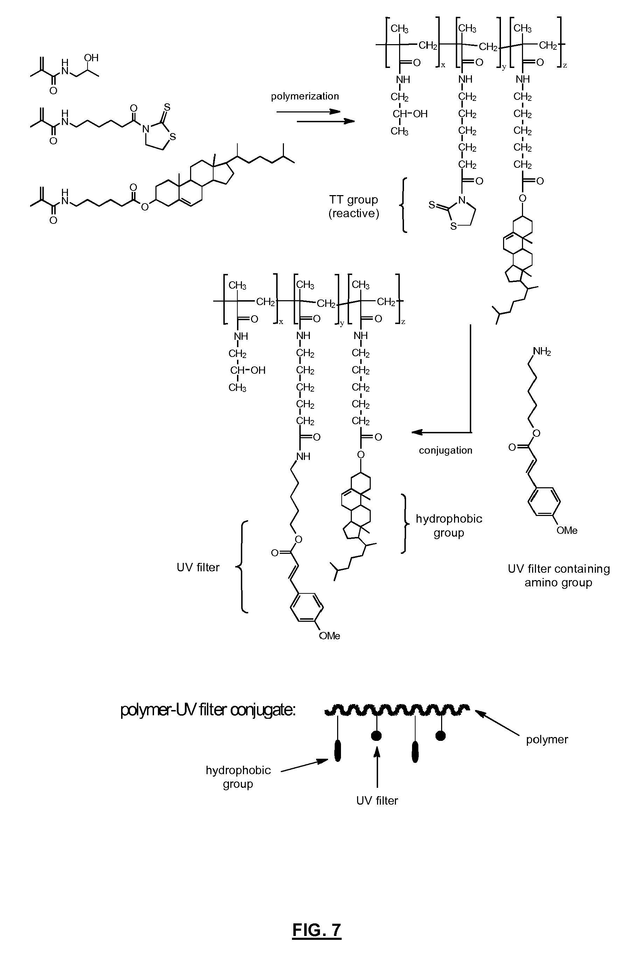

In a preferred embodiment, copolymers are obtained by free radical copolymerization of a mixture of hydrophilic and hydrophobic monomers in solution. Then, the compounds of the invention are synthesised by direct copolymerization (figure 6). Alternatively, the compounds of the invention can be synthesized by modification of preformed amphiphilic copolymers, particularly by the subsequent reaction of reactive polymers with drugs or drug derivatives having appropriate functional groups (figure 7). In a preferred embodiment, the amphiphilic copolymer is prepared from hydrophilic monomers, based on methacrylamides, and methacryloylated derivates of fatty acids, sterols, lipids, waxes or any other hydrophobic hydrocarbon (with a preferred number of carbons higher than 8) as the hydrophobic monomers. In a more preferred embodiment, the hydrophilic copolymer includes N-(2-hydroxypropyl)methacrylamide as the hydrophilic monomer and a methacryloylated derivative of cholesterol with the following general structure as the hydrophobic monomers: 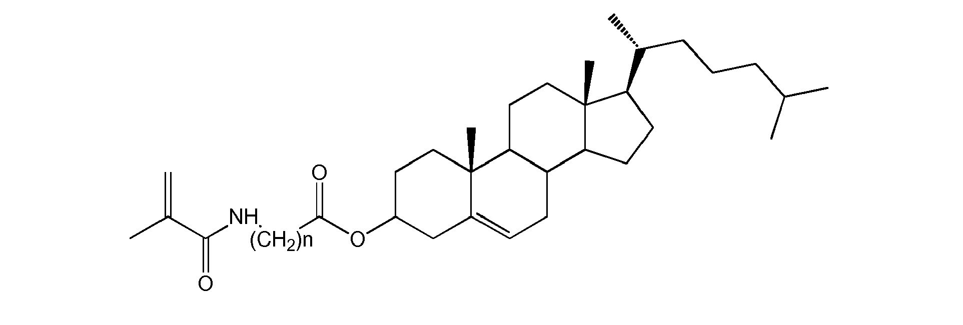 n=1-10

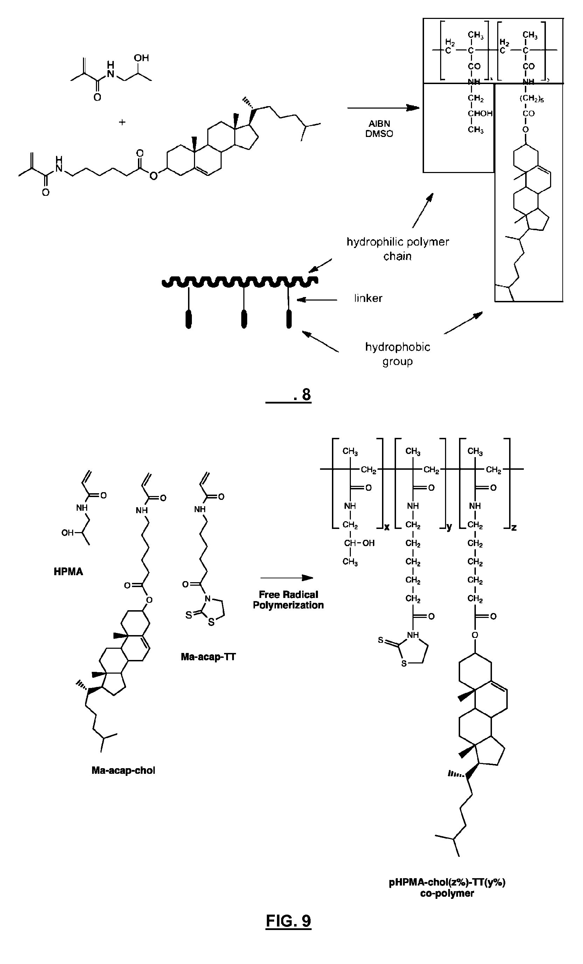

In a preferred embodiment, the copolymer includes about 90 to 99 mol wt% N-(2-hydroxypropyl)methacrylamide monomer and 1 to 10% mol wt% methacryloylated derivative of cholesterol (Ma-acap-chol, n = 5). These two particular monomers are selected partially because of their great difference in hydrophilicity, which will confer a strong amphiphilic character to the resulting copolymer. More particularly, N-(2-hydroxypropyl)methacrylamide (HPMA) is selected for its high water solubility. Methacryloylated derivative of cholesterol is selected because cholesterol is a natural component of the intercellular lipid matrix of the stratum corneum, and has the ability to intercalate into lipid bilayers. In fact, one of the natural main functions of cholesterol is to insert into lipid bilayers to regulate membrane fluidity. The final structure of the copolymer can be defined as a linear water-soluble polymer chain with pendant hydrophobic cholesterol groups connected by a short linker. The proposed copolymers have the general structure shown in figure 8.

In this respect, the inventors are of the view that, even though the below examples have been carried out with pHPMA as the preferred copolymer and a methacryloylated derivative of cholesterol as the preferred monomer having pendant hydrophobic groups, however the invention is not limited to these preferred compounds. On the contrary, any other copolymer and monomer having pendant hydrophobic groups could respectively be used in the present invention to the extent that they are able to fulfil their respective function as defined in the appended claims.

The copolymer components of the film-forming composition are preferably sufficiently small so that they mix well in solution and sufficiently large to provide efficient filming properties. It is believed that if the copolymer chains are too small, they will not provide the desired anchoring properties. In addition, if the copolymers are too large, it is believed that they will not result in homogeneous film formation. It is believed that when a copolymer is associated with the outermost layer of the skin, each inserted cholesteryl group has an additive effect, resulting in a co-operative effect that contributes to the stability of the whole anchored polymer. Coating layers formed by low molecular weight copolymer chains show greater desorption than coatings formed using higher molecular weight polymers. The higher stability of the latter polymers is explained by the greater length of their copolymer chains, which corresponds to a higher number of cholesteryl groups per polymer chain. The copolymers preferably have a number average molecular weight of less than 1,000,000 g/mol and more than 20,000 g/mol. More preferably copolymers have a number average molecular weight about greater than 50,000 g/mol and smaller than 500,000 g/mol, even more preferable greater than 70,000 g/mol and smaller than 200,000 g/mol, and most preferable about 100.000 g/mol.

In addition to hydrophobic and hydrophilic monomers, copolymers used in this invention can also contain monomers suitable for further labeling, post-polymerization modification and/or conjugation of active ingredients. Such monomers are designed as methacryloylated derivatives containing amines, carboxylic acids, hydrazones, thiols, hydroxyls, glycols, ketones, aldehydes, reactive groups, or any other functional group suitable for chemical conjugation of a molecule to the amphiphilic copolymer. In a preferred embodiment, the amphiphilic copolymer is prepared by polymerization of hydrophilic and hydrophobic monomers, and monomers bearing a reactive ester. Reactive ester groups allow post-modification of the copolymer with active ingredients bearing free amino groups. Among others, typical reactive ester groups include p-nitrophenyl esters, pentafluorophenyl esters, N-hydroxysuccinimide esters, thiazolidine-2-thione groups or 1-hydroxy-benzotraizole groups. Methods of synthesizing these reactive groups are well known to those skilled in the art, and are widely described in the literature.

In a preferred embodiment, the copolymer includes about 70 to 98 mol wt% HPMA monomer, 1 to 10 mol wt% methacryloylated derivative of cholesterol, and 1 to 20 mol wt% methacryoylated derivative of reactive thiazolidine-2-thione group (Ma-acap-TT). The proposed copolymers have the general structure shown in figure 9.

### Polymer coating of skin

In a recent publication (Chytil⁷), similar amphiphilic copolymers have been described in the literature. However, the compounds disclosed by Chytil must have a relatively low molecular weight, since they are intended to be eliminated from the body by renal filtration, so that they must be below the limit of renal filtration (about 50,000 g/mol). As a consequence, due to their relatively low molecular weight, those compounds cannot be multivalent, i.e. cannot have a multiplicity of hydrophobic pendant groups per copolymer chain.

Moreover, when dissolved in aqueous solutions, the amphiphilic copolymers disclosed by Chytil probably self-assemble into nanometric supramolecular structures, probably as polymeric micelles due to the amphiphilic nature of the copolymers. Therefore, they are not intended to bind onto the skin, but instead they are released to the blood in the hope that they accumulate over the tumours causing an enhanced antitumour activity. In contrast, in the present invention the copolymers are used as skin-bonding materials for the immobilization of active ingredients on the skin surface. In the presence of lipid-rich domains, such as the intercellular lipid matrix found in the stratum corneum, it is probable that the polymeric micelles reorganize their structure in the following manner: Hydrophobic interactions drive the pendant hydrophobic groups to insert into the intercellular lipid matrix, while the hydrophilic polymer backbone remains localized on the skin surface. On the contrary, the amphiphilic copolymer chains of the invention are multivalent, this means that copolymers have multiple hydrophobic pendant groups per chain. After a first pendant hydrophobic group of a copolymer chain inserts into the intercellular lipid matrix of the stratum corneum and anchors the copolymer chain to the skin, other pendant hydrophobic groups of the latter will insert into the stratum corneum, thereby immobilizing the copolymer chain through multiple hydrophobic interactions. Repeating hydrophobic skin-copolymer interactions result in stable anchoring of copolymer on the skin surface. Immobilization of multiple copolymer chains results in homogeneous polymer coating of the skin surface, leading to film formation.

As shown in example IV, increasing concentration of pendant hydrophobic groups per copolymer chain resulted in increasing film stability against wash off, when polymer coated skin was incubated in saline solutions. This behaviour suggests that each single pendant hydrophobic group-skin interaction contributes to the final stability of the polymeric film. Films prepared with amphiphilic copolymers bearing at least 5 mol% (or more) pendant hydrophobic groups showed complete stability against wash off, when incubated in saline solutions. These films are best suited for waterproof applications. In contrast, in films prepared with amphiphilic copolymers bearing lower concentrations (below 5 mol% but higher than 1 mol%) of pendant hydrophobic groups, a limited stability against wash off was observed, which renders them useful for other applications.

When a suitable preparation for topical application (e.g. liquid or a semisolid formulation) containing amphiphilic copolymers is applied to the skin surface and allowed to dry, a film is formed on the stratum corneum, even though sometimes this film is not completely continuous. Preferably, in all cases the skin is first washed with soap and water and thoroughly towelled dry before application of the film-forming preparation. The skin-bonding composition can be applied sparingly (this means, sufficiently to coat), extended over the skin surface and allowed to thoroughly dry for a few minutes. When the composition containing the amphiphilic copolymer dries on the skin, it forms a highly water-resistant film with cosmetically pleasing properties. It is believed that amphiphilic copolymers form stable films against sweat, water and extended wear, without inhibiting natural excretion of fluids from the skin. In addition, films are non-sticky and non-transferable, almost invisible and have attractive mechanical properties such as flexibility and abrasion resistance.

An advantage of the film-forming composition is that it can be provided without organic solvents that can be potentially harmful to the skin. In contrast, other film-forming compositions available on the market are based on water insoluble polymers, such as poly(alkyl-acrylate)s (Petkewich²¹) or cellulose acetate butanoate (Casper⁶), and require the use or organic solvents for their application. In general, such polymers, formulated as liquid compositions, are applied to the skin surface to produce water-insoluble films upon solvent evaporation. In contrast, the amphiphilic copolymers described in this invention are most preferably water-soluble, or, at most, require minimum amounts of alcohol (preferably ethanol or isopropanol) to be dissolved in hydroalcoholic preparations. Thereby, water resistance of the films prepared from amphiphilic copolymers is not caused by the water insolubility of the film forming materials. Instead, amphiphilic copolymers produce water-resistant films through multivalent copolymer-skin interactions that anchor the polymeric chains to the stratum corneum.

Furthermore, the present invention introduces an alternative way of delivering active ingredients to the skin surface, resulting in greater cosmetical attractiveness. In general, active ingredients used for dermal applications are hydrophobic and water insoluble. For this reason, organic solvents, waxes, petroleum distillates, oils and silicones are widely used in the preparation of topical preparations, in order to dissolve active ingredients and to apply them easily and evenly on the skin surface. However, such additives are sometimes occlusive to the skin, can interfere with normal skin transpiration and other physiological processes associated with healthy skin and/or produce a cosmetically unattractive effect on the skin. This invention provides a new way of delivering active ingredients to the skin surface by using topical preparations with lower contents of these potentially harmful or cosmetically unattractive additives. Conjugation of active ingredients to amphiphilic copolymers increases water solubility, when compared with free active ingredients. Thereby, copolymer conjugated active ingredients can be formulated using dermal compositions with higher proportion of aqueous components, without the need of complex or unpleasent organic additives.

### Conjugation of active ingredients to amphiphilic copolymers

### ● Stable linkage

In the present invention, an active ingredient is attached to the copolymer to obtain an active ingredient-copolymer conjugate. In this preferred embodiment, the attachment between the active ingredient and the copolymer is a stable, i.e. non-cleavable covalent linkage. The particular linkage can be any linkage that preserves the activity of the active ingredient. Preferably, such linkages result from reactions with reactive groups present on the copolymer. Other preferred groups on the copolymer are hydroxy, carboxy, thiol and amine groups. Examples of linkages include, but are not restricted to esters, amides, ethers, carbonates and ureas, among others. Preferred linkages are esters, ethers, carbamates and amides. Methods of synthesizing these linkages are well known to those skilled in the art. Such methods can be found, for example, in handbooks of synthetic organic chemistry, compendia such as "The Beilstein Handbook of Organic Chemistry" (Springer-Verlag Berlin, ISBN: 978-3540540908), "Reagents for Organic Chemistry" (Mary Fieser, Wiley-Interscience N.Y., ISBN: 978-0471000747), and "Advanced Organic Chemistry" (Jerry March, John Wiley & Sons N.Y., ISBN: 978-0471601807).

### ● Cleavable linkage

In another preferred embodiment, the active ingredient is attached to the copolymer in a reversible manner. In this preferred embodiment, the bond between the active ingredient and the copolymer can be cleaved through a trigger mechanism present on the skin surface. Following specific bond cleavage, the active ingredient is released from the copolymer onto the skin surface. Preferable degradation times are less than one month, more preferable degradation times are of the order of weeks, and most preferably days or hours.

Preferred trigger mechanisms include chemical features found in the skin or body fluids contained on the skin or chemical substances found in both. In a preferred embodiment, the trigger mechanism is the slightly acidic pH, between 4.5 and 5.5, found on the natural acid mantle of the skin (Wa³², Klee¹⁵). Then, the natural acid mantle of the skin can be used as an "automatic" trigger mechanism. In another preferred embodiment, active ingredients are released through the local low pH achieved through sweating or other exogenous means. In another preferred embodiment, active ingredients are released by the action of naturally occurring enzymes present on the skin surface. In another preferred embodiment, active ingredients are released by the action by enzymes produced by microorganisms present on the skin (Sondell²⁷).

The particular linkage that connects the active ingredient and the amphiphilic copolymer in a reversible manner can be any linkage that does not alter the activity of the active ingredient upon release. Preferably, such linkages are stable under the conditions found in the topical preparation, in order to minimize release during storage conditions and ensure selective release upon dermal application. In addition, the release kinetics of the active ingredients from the copolymer must be compatible with the demand of free active ingredient needed in order to correctly perform its therapeutic or other activity. The type of linkage used to conjugate the active ingredient to the amphiphilic copolymer depends on the trigger mechanism and the suitable functional groups found on the active ingredient. The release of active ingredients, especially drugs, from polymers and other macromolecular structures has been widely studied during the last decades (Etrych¹¹, Cameron³⁵). Extensive research has led to the development of a great number of triggered-release mechanisms that can be adapted to the amphiphilic copolymers described in this invention (see example VII). Methods of synthesizing these linkages are well known to those skilled in the art and have been extensively reviewed in the literature (Ulbrich³¹). The following table (table 1) summarizes some of the conjugation strategies that are compatible with the three main trigger mechanisms found on the skin surface: pH, enzymatic activity and light.

**Table 1. Trigger mechanisms and conjugation strategies to reversibly immobilize active ingredients on amphiphilic copolymers:**

| **Trigger mechanism** | **Type of bond** | **Chemical functionality on active ingredient** |
|---|---|---|
| **pH** | hydrazone | ketone, aldehyde |
| | acetal, ketal | ketone, aldehyde |
| | mixed acetal - ketal | hydroxyl |
| | orthoester | hydroxyl |
| | imine | amine |
| **enzyme** | amide (peptide) | amine |
| | ester | hydroxyl |
| **light** | 2-nitrobenzy ester | hydroxyl |

In another preferred embodiment, the dermal composition combines free and polymer-conjugated active ingredient resulting in a complex formulation with a sustained release profile. In such systems, free active principle sets the starting dose, while polymer-conjugated active principle helps to prolong the initial dose by slowly liberating free active principle.

### Active ingredients

As previously described, films prepared from amphiphilic copolymers immobilized on the skin surface are sufficiently substantial to work as reservoirs of active ingredients. The formulations of the present invention can include a great number of polymer conjugated active ingredients due to the suitability of the conjugation chemistry. Examples of active ingredients preferred in this invention include humectants, sunscreens, fragrances, insect repellents, pigments, and cosmetically or dermatologically active agents and drugs.

### Humectants

A preferred use of the film-forming compositions of the present invention is as a long lasting humectant. Humectants are a key ingredient in moisturizers and benefit the skin by increasing the water content in the epidermis. Among other features, humectants can increase the pliability and flexibility of the skin, prevent cracking, reduce skin irritation caused by dry skin conditions and prevent outside chemicals from contacting the dermis. Moisturizers not only increase the skin's water content, but they also protect the skin and encourage an orderly desquamation (shedding) process that makes the skin appear smoother. A number of humectants are known, such as acetamide MEA, agarose, ammonium lactate, arginine PCA, betaine, butylene glycol, copper PCA, corn glycerides, diglycereth-7 malate, diglycerin, dimethyl imidazolidinone, erythritol, gelatin, glucose, glucuronic acid, glucuronolactone, glutamic acid, glycereth-12, glycerin, hyaluronic acid, hydroxyethyl sorbitol, lactamide lactic acid, maltitol, melibiose, panthenol, pantolactone, 2-pyrrolidone-5-carboxylic acid, polyglucuronic acid, propylene glycol, sodium aspartate, sodium lactate, sodium malate, sodium 2-pyrrolidone-5-carboxylate, sodium polyaspartate, sorbitol, TEA lactate, triglycereth-7 citrate, urea, N-(2-hydroxyethyl)urea, xylose and mixtures thereof.

The amphiphilic copolymers described herein already have humectant properties *per se,* as shown in experiment VIII. Films prepared from amphiphilic copolymers deposited on an inert and permeable membrane were able to absorb and retain water from a controlled atmosphere at a fixed relative humidity. In a preferred embodiment, amphiphilic copolymers are preferably conjugated with at least one humectant compound of the list described above, in order to increase the humectant ability of the copolymer. When applied on the skin surface as a suitable preparation, a stable film with humectant properties can be formed.

### Sunscreen

Another preferred use of the film-forming compositions of the present invention is as a long lasting sunscreen carrier. Skin cancer has become one of the fastest growing cancers and its incidence is closely related to sun exposure (Wang³³). Especially, two types of radiation, UVA and UVB, are involved in the rise of skin cancer occurrence during the last years. In order to reduce the risk of developing skin cancer, it is recommended to reduce direct sun exposure or, if this is unavoidable, to use sun protection measures, such as sunscreens containing appropriate UV filters. UV filters are classified according to their nature, either as inorganic particulates or organic UV-absorbing molecules. Inorganic particulates, such as metal oxides, protect the skin by reflecting harmful UV radiation. In contrast, organic UV filters are designed to absorb certain types of radiation of the UV spectrum.

A number of approved sunscreen active ingredients with UV filtering properties are known, such as, among others, p-aminobenzoic acid (PABA), 2-ethylhexyl 4-dimethylaminobenzoate (Padimate O), Phenylbenzimidazole sulfonic acid, Cinoxate, Dioxybenzone, Oxybenzone, Homosalate, Menthyl anthranilate, Octocrylene, 2-ethylhexyl 4-methoxycinnamate, Isoamyl p-Methoxycinnamate, 2-ethylhexyl 4-salicylate, Sulisobenzone, Trolamine salicylate, Avobenzone, Ecamsule, 4-Methylbenzylidene camphor, Tinosorb S, Tinosorb M, Bisdisulizole disodium (Neo Heliopan AP, Mexoryl XL, Benzophenone-9, Uvinul T 150, Uvinul A Plus, Uvasorb HEB, Parsol XLS, Titanium dioxide, Zinc oxide and mixtures thereof.

In general, sunscreen active ingredients are water insoluble. For this reason, pharmaceutical and cosmetic preparations employ the use of alcohols, waxes, petroleum distillates, oils and silicones to dissolve sunscreens and keep them on the skin. However, these ingredients are occlusive to the skin and do not promote healthy skin chemistry. In general, extended wear, water-sport activities and/or sweat lead to decreased efficiency of the sun blocking activity, due to wash off of the active principles from the skin. For this reason, all sunscreen formulations require repeated application after swimming or sweating. In addition to wash off, percutaneous absorption of UV filters has also been observed in many sunscreen preparations. Transdermal permeation of UV filters is a major disadvantage since it directly decreases the UV blocking efficiency of sunscreen products. In addition, permeation of UV absorbing substances into systemic circulation can result in body accumulation, leading to potential health risks (Hayden¹²). Attempts to increase the residence of UV filters on the skin and avoid percutaneous absorption include new formulation forms, such as nanoparticles (Alvarez-Roman³) and polymers (Patanaargson²⁰). Both nanoparticle encapsulation and polymer conjugation of UV absorbing compounds have shown to avoid skin penetration, probably due to the low diffusion of these molecular macrostructures through the skin.

In a preferred embodiment, amphiphilic copolymers are conjugated with at least one UV blocking material of the list described above. When applied on the skin surface, a stable and homegeneous film with UV blocking properties is formed. Water-resistant films having UV blocking activity are especially suitable for extended wear and water-sport activities, in order to reduce wash off of the UV absorbing molecules from the skin. In example VI, the film-forming composition described herein was coupled with an effective UV absorbing agent, p-methoxycinnamate ester. The UV absorbing ability of the cinnamate ester was not significantly changed after copolymer conjugation.

### Fragrance

Another preferred use of the film-forming compositions of the present invention is as a long lasting fragrance, either as a way of releasing a pleasant odour over a prolonged period of time or as a long lasting insect repellent. Fragrance preparations are prepared from intense-smelling and volatile molecules that usually contain alcohol, aldehyde, ketone and/or terpene functional groups. However, when applied on the skin, many of these compounds can only be perceived for a short period of time, due to their high volatility. The flavor and fragrance industry has overcome this limitation by using techniques based on the inclusion of active ingredients into matrixes, in order to prolong the long-lasting effect of volatile compounds (Liu¹⁷). Another important approach to prolong the fragrance effect involves the use of so called profragrances, which are precursor molecules that release active fragrance molecules upon cleavage of a chemical bond. Several trigger mechanisms, such as light, enzymes or pH (Saint Laumer²²), have been explored in order to chemically transform the precursor into an active fragrance molecule. Ideally, profragrances are odourless and non-volatile (or have decreased volatility), serve as protecting groups and increase the substantivity of the active fragrance. During the last years, new and more complex systems for the sustained and/or controlled release of fragrances have been developed by the fragrance industry, in order to increase the performance of perfumery products.

A preferred use of this invention is the development of polymeric - profragrance conjugates able to form stable films on the skin surface. Chemical cleavage of the bond connecting the amphiphilic copolymer and the profragrance can allow the release of the fragrance's active compound from the skin-immobilized films. For this purpose, profragrances have to be chemically conjugated to the amphiphilic copolymer via a reversible bond that can be selectively cleaved via a trigger mechanism found on the skin surface. Polymer conjugation of profragrances results in high molecular weight macromolecular structures that are odorless and non-volatile. As previously described, films prepared from amphiphilic copolymers are highly stable and cosmetically attractive, which made them especially suitable for immobilizing precursor fragrances for extended and/or triggered release applications in a broad variety of perfumery and personal care applications.

A large number of fragrance compounds have been obtained by the fragrance industry, etiher from natural sources or through synthetic approaches. Typical compounds used in the fragrance industry are well known to those skilled in the art. Such compounds can be found, for example, in textbooks of fragrance and flavor chemistry, such as "Perfume and Flavor Chemicals" (S. Arctander, Allured Publishing, ISBN 0-931 71 0-35-5). Examples of fragrance compounds include, but are not restricted to: isoamyl acetate, myrcene, limonene, anisole, eugenol, cinnamaldehyde, vanillin, alpha-ionone, camphor, citral, citronellol, geraniol, fructone, jasmone, linalool, muskone, damascone, menthone, lyral, 1-decanal, nerol, 1-hexanal, thymol, farnesol or citronellal, among others.

In the present invention, preferred fragrance compounds contain ketone, aldehyde and/or alcohol functional groups on their chemical structure, so that fragrance molecules can be chemically conjugated to the amphiphilic copolymers via these functional groups. In this preferred use, the bond between the fragrance molecule and the copolymer can be cleaved through a trigger mechanism present on the skin surface, as previously described. Preferred chemical conjugation strategies compatible with these functional groups, include hydrazone, acetal, ketal, orthoester and ester formation.

### Cosmetic and dermatologic active agents & drugs

Preferred cosmetic and dermatologic active ingredients include biologically active ingredients that are applied topically to improve skin health and attractiveness. Preferred active ingredients include compounds with antioxidant, free radical scavenger, antiage, anti-inflammatory, keratolytic, whithening, slimming/anti-cellulite, wrinkle repair, lifting, firming, skin toning, oil control and hair growth modulation properties. Preferred cosmetic active ingredients include, but are not restricted to, vitamins, alpha-hydroxyacids, oligopeptides, co-enzymes, co-factors, caffeine, UV filters, cutaneous protection agents, colorants, pigments, pigment modulators, retinoids, and antioxidants, among others. In a preferred embodiment, active ingredients are conjugated to an amphiphilic copolymer via a chemical bond that is stable at the physiological conditions found on the skin surface. When applied to the skin surface, polymer active ingredient conjugates form stable films avoiding wash off and percutaneous aborption. In another preferred embodiment, active ingredients are conjugated to an amphiphilic copolymer via a chemical bond that can be cleaved through a trigger mechanism present on the skin surface. When applied to the skin surface, polymer - active ingredient conjugates form stable films that work as a reservoir of active ingredients for extended and/or controlled release applications. Following specific bond cleavage, active molecules are released from the copolymer onto the skin surface to perform their beneficial action.

Preferred drugs include compounds that are normally applied topically and used to treat or ameliorate skin diseases or medical skin conditions, such as infections, allergies, inflammation episodes, pigmentation disturbances and other conditions that may alter the normal function of the skin. Preferred drugs include, but are not restricted to, active ingredients with antibiotic, antifungicide, anti-inflammatory, anti-itching, soothing, antiseptic, anti-acne, anti-psoriatic, anti-rosacea, anti-melasma, antiviral, hormones, cutaneous growth enhancers, topical anesthetics, phototherapeutic agents, chemotherapeutic agents or any other therapeutically benefitial properties. In a preferred embodiment, drugs are conjugated to an amphiphilic copolymer via a chemical bond that can be cleaved through a trigger mechanism present on the skin surface. When applied to the skin surface, polymer - drug conjugates form stable films that work as a drug reservoir for extended and/or controlled release applications. Following specific bond cleavage, drug molecules are released from the copolymer onto the skin surface to perform their therapeutic action.

In another preferred embodiment, the active ingredient is attached to the copolymer in a reversible manner. In this preferred use, the bond between the active ingredient and the copolymer can be cleaved through a trigger mechanism present on the skin surface. Following specific bond cleavage, the active ingredient is released from the copolymer onto the skin surface.

The invention will now be described in more detail by reference to the following examples. The only proper construction of these examples is as non-limiting illustrative examples showing various formulations, stabilities, and applications of the invention.

### Examples

### Example I

### ● Synthesis of Monomers

Synthesis of monomers was performed on a multigram scale following previously published procedures or adaptations of them. Methacryloyl chloride was freshly distilled before every use. Detailed synthesis and characterization for each monomer is described below:

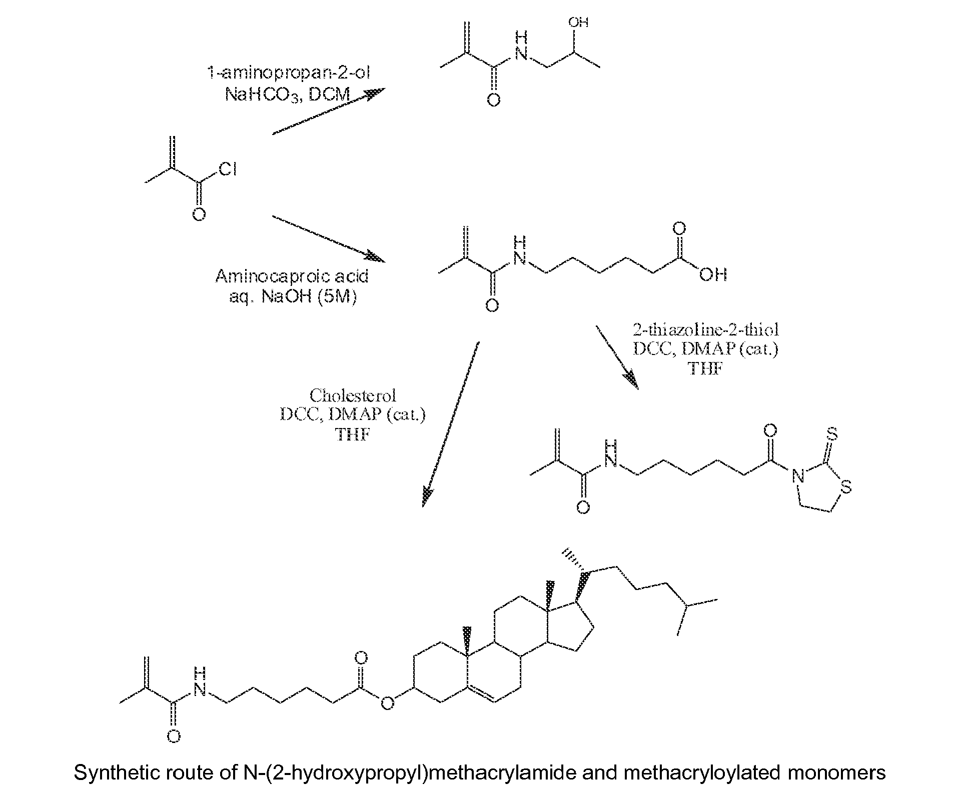

### ● Synthesis of N-(2-hydroxypropyl)methacrylamide (HPMA)

N-(2-hydroxypropyl)methacrylamide, HPMA, monomer was synthesized by acylation of 1-aminopropan-2-ol with methacryloyl chloride, using anhydrous sodium hydrogen carbonate (NaHCO₃) as base to quench the HCl byproduct evolved during the reaction (Ulbrich³⁰).

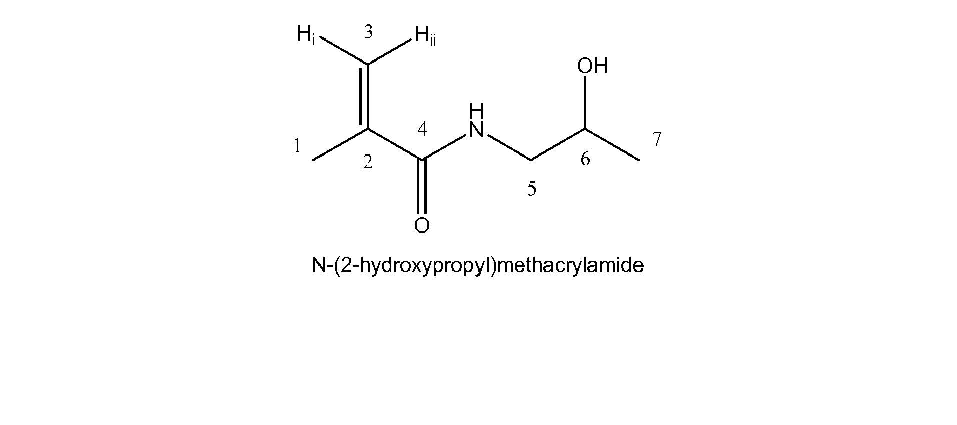

To a cooled (-20°C) stirred suspension of anhydrous sodium hydrogen carbonate (12.5 g, 0.15 mmol) and 1-aminopropan-2-ol (8.5 g, 0.11 mmol) in anhydrous DCM (40 mL) was added drop-wise a solution of methacryloyl chloride (11.8 g, 11.0 mL, 0.11 mmol) in anhydrous DCM (20 mL) over 1 hour. The reaction mixture was stirred for a further hour at -20°C and for another 30 min at room temperature. The reaction mixture was dried with Na₂SO₄ (anhyd.), concentrated *in vacuo* to approximately 25 mL and cooled to -20°C. A white precipitate was isolated and recrystallized from Et₂O:MeOH (3:1) yielding a white crystalline solid (yield 6.1 g, 39%).
**¹H-NMR** (200 MHz, CDCl₃, TMS) (ppm): δ = 6.63 (*s*, 1H, N*H*), 5.71 (*s*, 1H, H-3ᵢᵢ), 5.32 (*t*, 1H, H-3ᵢ), 3.91 (*m*, 1H, H-6), 3.70 (*d*, 1H, O*H*), 3.46 (*dq*, 1H), 3.13 (*m*, 1H), 1.94 (*s*, 3H, H-1), 1.16 (*d*, 3H, H-7)
**¹³C-NMR** (200 MHz, CDCl₃, TMS) (ppm): δ = 169.62 (C-4), 140.04 (C-2), 120.52 (C-3), 67.54 (C-6), 47.58 (C-5), 21.33 (C-7), 19.07 (C-1)
**IR** (ATIR) ν = 659, 824, 845, 914, 1001, 1053, 1088, 1117, 1142, 1232, 1263, 1331, 1427, 1553, 1614, 1653, 2933, 2976, 3275, 3306 cm⁻¹
MS (ESI) m/z = 142 [M-H]
EA C = 58.72% (exp. 58.93%), H = 9.15% (exp. 9.48%), N = 9.78% (exp. 9.83%)

### ● Synthesis of N-methacryloyl-6-aminohexanoic acid (Ma-acap-OH) Ma-acap-OH was synthesized by acylation of aminocaproic acid with methacryloyl chloride in aqueous NaOH, in a procedure known as the Schotten-Baumann reaction.

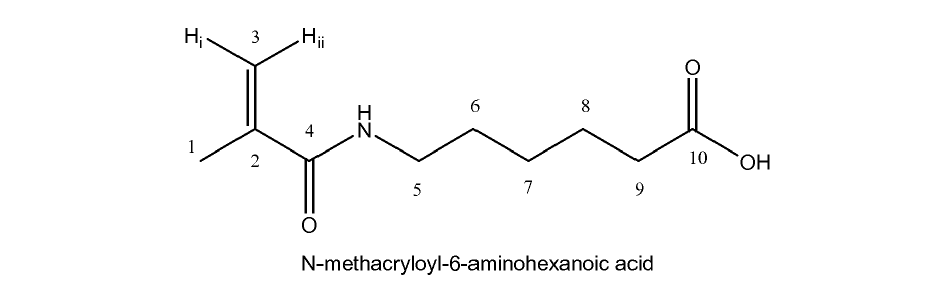

To an ice cooled stirred solution of sodium hydroxide (5.2 g, 0.12 mol) and aminocaproic acid (16.9 g, 0.12 mol), in water (26 mL) was added drop-wise methacryloyl chloride (13.4 g, 0.13 mol) and sodium hydroxide (5.2 g, 0.12 mol) over 20 minutes. The reaction mixture was stirred for a further hour at room temperature. The reaction mixture was extracted with 25 mL DCM. The aqueous portion was cooled using ice and then acidified under a layer of EtOAc (150 mL) to pH 2.0 using 6.9 M HCl. The organic phase was removed and the aqueous layer extracted using 3 x 75 mL EtOAc. The organic portions were then combined, dried with NaSO₄ (anhyd.) and concentrated *in vacuo* to 75 mL. Et₂O (75 mL) was added to the solution and cooled to -20°C. A white precipitate was isolated and recrystalized twice from EtOAc:Et₂O (1:1) yielding a white crystalline solid (yield 11.8 g, 46%). Mp: 54-55°C (Subr³⁶).
**¹H-NMR** (200 MHz, CDCl₃, TMS) (ppm): δ = 10.52 (*s*, 1H, COOH), 6.19 (*s*, 1H, N*H*), 5.67 (*s*, 1H, H-3ᵢᵢ), 5.29 (*t*, 1H, H-3ᵢ), 3.28 (*q*, 2H, H-5), 2.33 (*t*, 2H, H-9), 1.92 (*s*, 3H, H-1), 1.70 - 1.31 (*m*, 6H, H-6 H-7 H-8)
**¹³C-NMR** (200 MHz, CDCl₃, TMS) (ppm): δ = 178.70 (C-10), 169.38 (C-4), 140.24 (C-2), 120.21 (C-3), 39.93 (C-5), 34.32 (C-9), 29.47, 26.70, 24.70, 19.07 (C-1).
**IR** (ATIR): ν = 679, 722, 928, 1196, 1232, 1279, 1329, 1423, 1429, 1458, 1531, 1605, 1651, 1693, 2880, 2943, 3335 cm⁻¹

### Synthesis of methacryloylated active ester (Ma-acap-TT)

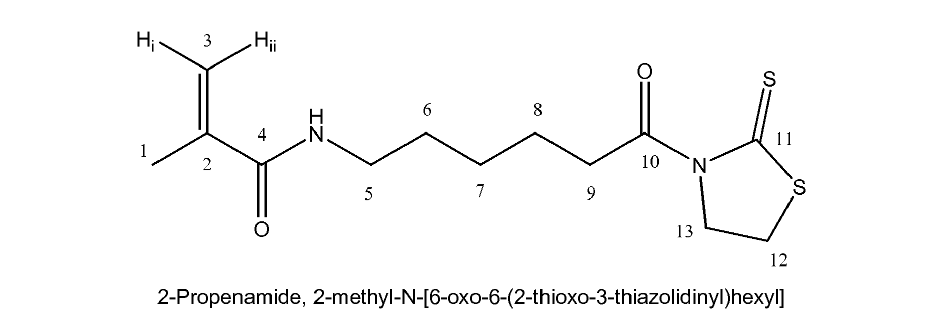

To a cooled solution (-20°C) of N-methacryloyl-6-aminohexanoic acid (3.0 g, 0.015 mol) and 2-thiazoline-2-thiol (1.8 g, 0.015 mol) in dry THF (35 mL) was added a cooled solution (-20°C) of N,N'- dicyclohexylcarbodiimide (DCC) (3.72 g 0.018 mol) in dry THF (5 mL). Some crystals of DMAP were added and the reaction mixture was kept for 1 h at -20°C and overnight at 5°C. The reaction mixture was stirred for a further hour at room temperature, acetic acid (0.1 mL) was added and stirred for another 30 min. The reaction mixture was filtered (to remove N,N'-dicyclohexylurea, DCU) and concentrated *in vacuo.* The resulting oil was dissolved in EtOAc (50 mL) and the solution filtered. The solution was concentrated *in vacuo* and the resulting oil was dissolved in Et₂O (50 mL) and cooled at -20°C. A pale yellow precipitate was isolated and recrystallized from Et₂O yielding a yellow crystalline solid (yield 2.5 g, 56%). Mp: 59-62°C
**¹H-NMR** (200 MHz, CDCl₃, TMS) (ppm): δ = 5.86 (*s*, 1H, N*H*), 5.67 (*s*, 1H, H-3ᵢᵢ), 5.31 (*t*, 1H, H-3ᵢ), 4.58 (*t*, 2H, H-13), 3.29 (*m*, 6H, H-5, H-9, H-12), 1.97 (*s*, 3H, H-1), 1.80 - 1.30 (*m*, 6H, H-6 H-7 H-8)
**¹³C-NMR** (200 MHz, CDCl₃, TMS) (ppm): δ = 202.10 (C-11), 174.99 (C-10), 168.83 (C-4), 140.59 (C-2), 119.67 (C-3), 56.48 (C-13), 39.79 (C-5), 38.73 (C-9), 29.47, 28.78 (C-12), 26.70, 24.70, 19.07 (C-1).
**IR** (ATIR) ν = 677, 717, 878, 933, 1005, 1039, 1150, 1232, 1279, 1356, 1387, 1549, 1605, 1651, 1697, 2855, 2930, 3284 cm⁻¹

### ● Synthesis of methacryloylated cholesterol (Ma-acap-chol)

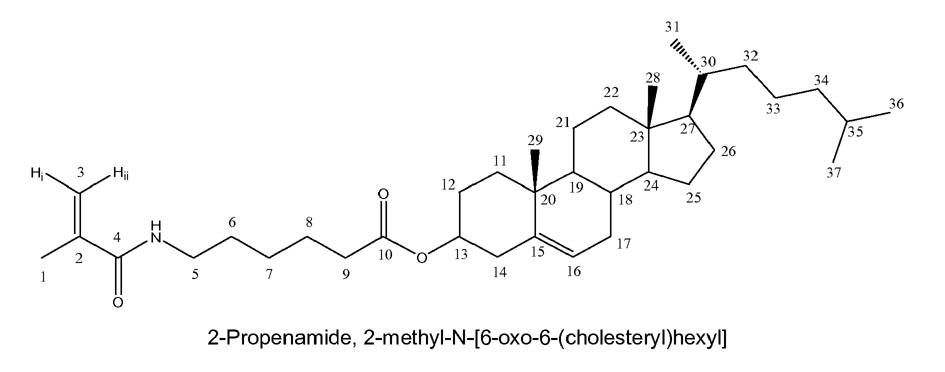

To a cooled (-20°C) stirred solution of N-methacryloyl-6-aminohexanoic acid (1 g, 0.005 mol) and cholesterol (1.934 g, 0.005 mol) in dry THF (15 mL) was added a cooled solution (-20°C) of DCC (1.24 g, 0.006 mol) in dry THF (3 mL). The reaction mixture was stirred for a further hour at room temperature, acetic acid (0.1 mL) was added and stirred for another 30 min. The reaction mixture was filtered and concentrated *in vacuo.* The resulting oil was dissolved in EtOAc (50 mL) and the solution was filtered again. The reaction mixture was washed with sodium bicarbonate (2%, 2 x 20 mL) to remove the unreacted N-methacryloyl-6-aminohexanoic acid and the organic layer was dried with anhydrous MgSO₄. The solution was concentrated *in vacuo* and the resulting oil was dissolved in acetone (25 mL) and cooled at -20°C (Chytil⁷). A white precipitate was isolated and recrystallized from acetone yielding a white crystalline solid (yield 1.6 g, 57%). Mp: 103-105°C
**¹H-NMR** (200 MHz, CDCl₃, TMS) (ppm): δ = 5.86 (*s*, 1H, N*H*), 5.67 (*s*, 1H, H-3ᵢᵢ), 5.37 (*d*, 1H, H-16), 5.31 (*t*, 1H, H-3ᵢ), 3.32 (*q*, 2H, H-5), 2.29, (*t*, 2H, H-14), 2.0 - 0.8 (complex signal, nH, methylene envelope), 0.67 (s, 3H, H-28)
**¹³C-NMR** (200 MHz, CDCl₃, TMS) (ppm): δ = 173.47 (C-10), 168.84 (C-4), 140.65 (C-15), 140.07 (C-2), 123,70 (C-16), 119.60 (C-3), 74.28 (C-13), 57.11, 56.56, 50.45, 42.74, 40.15, 39.95, 39.81, 37.41, 37.02, 36.61, 36.33, 34.87, 32.28, 29.64, 28.66, 28.44, 28.24, 26.77, 24.97, 24.71, 24.26, 23.26, 23.00, 21.46, 19.75, 19.15, 12.29.
**IR** (ATIR) ν = 731, 926, 1011, 1167, 1238, 1294, 1377, 1468, 1545, 1612, 1655, 1734, 2868, 2934, 3296 cm⁻¹

### ● Synthesis of Amphiphilic Copolymers

Amphiphilic copolymers were prepared by free radical polymerization following adapted procedures described in the literature. Cholesteryl bearing copolymers were prepared by direct copolymerization of HPMA and cholesteryl monomers (Ma-acap-chol). Monomers of methacryloylated active esters were added to the polymerization mixture in order to allow labelling and/or conjugation of active agents to the resulting co-polymer. The amount of active ester in the reaction mixture was fixed at 10 mol wt%. A range of pHPMA-chol-TT copolymers bearing different concentrations of cholesterol pendant groups was synthesized. The content of TT groups in the synthesized copolymers was determined by UV spectrophotometry using the Lambert-Beer law. The content of cholesterol-derivative groups in the synthesized co-polymers was determined by comparisson of integral intensities in ¹H-NMR spectrum. Average molecular weight of synthesized copolymers was determined by either Matrix-Assisted Laser Desorption/lonization Time-of-Flight (Maldi-Tof) mass spectrometry or Gel Permeation Chromatography using pHPMA standards of known molecular weight. Detailed synthesis and characterization for each copolymer is described below:

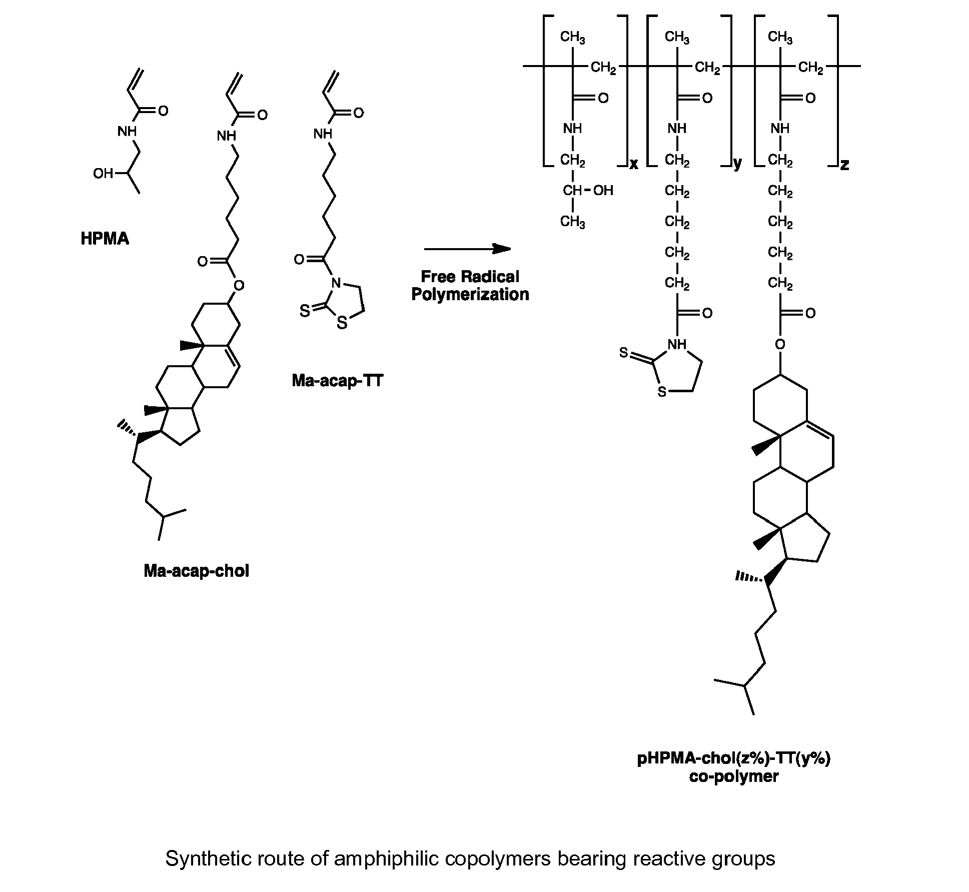

### ● Synthesis of pHPMA-chol(1%)-TT(10%)

N-(2-hydroxypropyl)methacrylamide (0.50 g, 3.49 mmol), Ma-acap-TT (0.118 g, 0.39 mmol), Ma-acap-chol (0.022 g, 0.04 mmol) and 2,2'-Azobis(2-methylpropionitrile) (AIBN) (0.102 g, 0.62 mmol) were dissolved in anhydrous DMSO (4.38 g, 4.0 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.36 g of a pale yellow solid.
Concentration of TT reactive groups in the copolymer: 6.4 mol%
Concentration of cholesterol groups in the copolymer: 1.4 mol%, equivalent to an average of 4.8 cholesterol groups per polymer chain.
Average molecular weight of the copolymer: 55,600 g/mol

### ● Synthesis of pHPMA-chol(2.5%)-TT(10%)

N-(2-hydroxypropyl)methacrylamide (0.50 g, 3.49 mmol), Ma-acap-TT (0.120 g, 0.40 mmol), Ma-acap-chol (0.057 g, 0.1 mmol) and 2,2'-Azobis(2-methylpropionitrile) (AIBN, 0.108 g, 0.66 mmol) were dissolved in anhydrous DMSO (4.63 g, 4.2 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.31 g of a pale yellow solid.
Concentration of TT reactive groups in the copolymer: 6.7 mol%
Average molecular weight of the copolymer: 55,500 g/mol
Concentration of cholesterol groups in the copolymer: 3.0 mol%, calculated as the percentage of monomers having at least one pendant cholesterol group. Since the average molecular weight of a monomer like pHPMA-chol(2.5%)-TT(10%) is 169.5 g/mol, then the average number of monomers contained in a molecule weighing 55,500 g/mol is about 327.5 monomers. Therefore, a concentration of 3.0 mol% of cholesterol groups in the copolymer is equivalent to an average of 9.8 cholesterol groups per polymer chain.

### ● Synthesis of pHPMA-chol(5%)-TT(10%)

N-(2-hydroxypropyl)methacrylamide (0.50 g, 3.49 mmol), Ma-acap-TT (0.123 g, 0.41 mmol), Ma-acap-chol (0.12 g, 0.21 mmol) and 2,2'-Azobis(2-methylpropionitrile) (AIBN) (0.118 g, 0.72 mmol) were dissolved in anhydrous DMSO (5.06 g, 4.6 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.26 g of a pale yellow solid.
Concentration of TT reactive groups in the copolymer: 6.6 mol%
Average molecular weight of the copolymer: 70,600 g/mol
Concentration of cholesterol groups in the copolymer: 5.4 mol%, equivalent to an average of 21.2 cholesterol groups per polymer chain

### ● Synthesis of pHPMA-chol(1%)-TT(15%)

N-(2-hydroxypropyl)methacrylamide (0.50 g, 3.49 mmol), Ma-acap-TT (0.19 g, 0.62 mmol), Ma-acap-chol (0.024 g, 0.04 mmol) and 2,2'-Azobis(2-methylpropionitrile) (AIBN) (0.11 g, 0.69 mmol) were dissolved in anhydrous DMSO (4.9 g, 4.4 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.26 g of a pale yellow solid.
Concentration of TT reactive groups in the copolymer: 11.9 mol%
Average molecular weight of the copolymer: 149,000 g/mol
Concentration of cholesterol groups in the copolymer: 1.1 mol%, equivalent to an average of 9.6 cholesterol groups per polymer chain

### ● Synthesis of pHPMA-chol(5%)-TT(15%)

N-(2-hydroxypropyl)methacrylamide (0.50 g, 3.49 mmol), Ma-acap-TT (0.2 g, 0.65 mmol), Ma-acap-chol (0.13 g, 0.22 mmol) and 2,2'-Azobis(2-methylpropionitrile) (AIBN) (0.13 g, 0.8 mmol) were dissolved in anhydrous DMSO (5.6 g, 5.1 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.26 g of a pale yellow solid.
Concentration of TT reactive groups in the copolymer: 11.5 mol%
Average molecular weight of the copolymer: 107,000 g/mol
Concentration of cholesterol groups in the copolymer: 5.2 mol%, equivalent to an average of 29.6 cholesterol groups per polymer chain.

### ● Synthesis of pHPMA-chol(0%)-TT(10%)

N-(2-hydroxypropyl)methacrylamide (0.50 g, 3.49 mmol), Ma-acap-TT (0.117 g, 0.39 mmol) and 2,2'-Azobis(2-methylpropionitrile) (AIBN) (0.099 g, 0.60 mmol) were dissolved in anhydrous DMSO (4.22 g, 3.8 mL). The reaction mixture was purged with Ar, sealed and placed in a pre-heated water-bath at 50 °C. After 6h, the reaction was stopped by taking the reaction vessel out of the water-bath, cooled down to room temperature. The copolymer was isolated by precipitation in a mixture of acetone:Et₂O (1:1) and dried under vacuum until constant weight, yielding 0.38 g of a pale yellow solid.
Concentration of TT reactive groups in the copolymer: 5.1 mol%
Average molecular weight of the copolymer: 50,600 g/mol
N.B.: For labelling purposes, % of TT and cholesterol groups in the copolymer are noted as theoretical values.

### ● Fluorescent labelling of pHPMA-chol(2.5%)-TT(10%) copolymer

One of the copolymers described above, pHPMA-chol(2.5%)-TT(10%) copolymer, was labelled with a fluorescent dye in order to evaluate the polymer behaviour *in vitro* using fluorescence microscopy techniques. Labelling of the copolymer was achieved by aminolysis of TT reactive groups with Alexa Fluor® 488 cadaverine sodium salt, as shown in the following scheme:

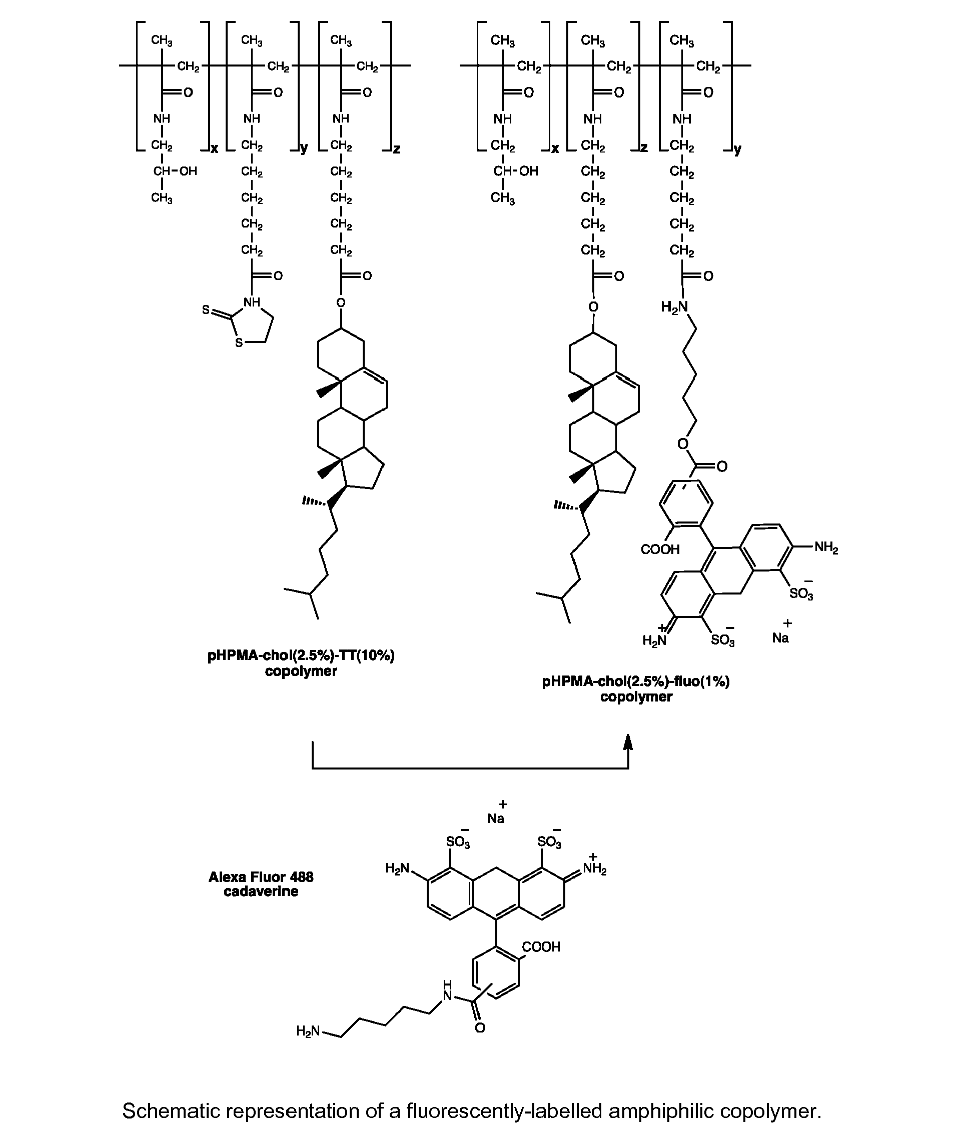

Copolymer labelling was calculated to achieve a final modification not exceeding 1 mol% to ensure minimal interference of the fluorescent label with the polymer backbone.

To a polymer solution (20 mg in 250 µL) in dry DMSO was added Alexa Fluor® 488 cadaverine sodium salt (50 µL) dissolved in anhydrous DMSO (1 mg/mL). The reaction mixture was stirred for 1 hour. The reaction was terminated by addition of aminopropan-2-ol (5 µL). The reaction mixture was stirred for 15 more minutes before precipitation into a mixture acetone:Et₂O (1:1, 60 mL). The copolymer was isolated by filtration, washed extensively with a acetone:Et₂O mixture (2:1) and dried under vacuum. The resulting copolymer was kept protected from light at 4°C.

### ● Labelling of amphiphilic copolymers with methyl red dye

The synthesized copolymers were labelled with methyl red dye in order to visualize the copolymer behaviour *in vitro.* Labelling of the copolymer was carried out by aminolysis of TT reactive-ester groups with an amino-derivative of methyl red, as shown in the following scheme:

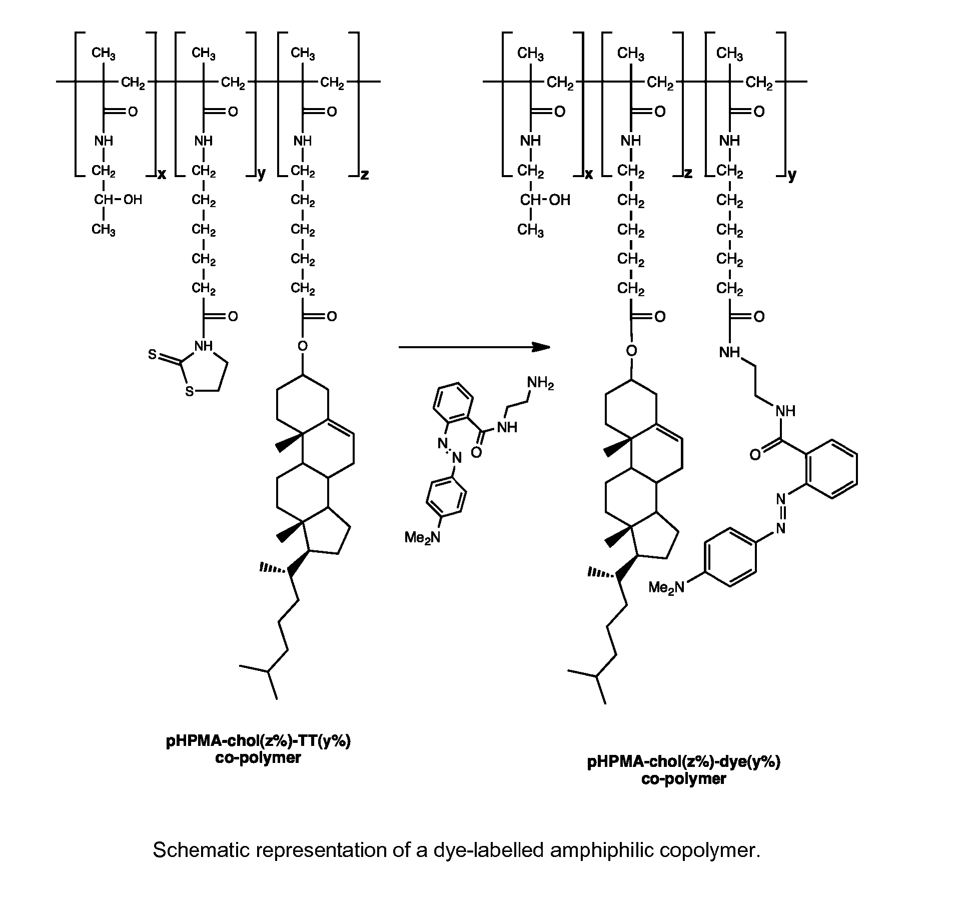

Copolymer labelling was calculated to achieve a final modification not exceeding 5 mol%.

To a polymer solution of pHPMA-TT(10%), pHPMA-chol(1%)-TT(10%), pHPMA-chol(2.5%)-TT(10%) or pHPMA-chol(5%)-TT(10%) (25 mg in 250 µL) in HPLC grade methanol was added an amino-derivative of methyl red (3 mg) dissolved in HPLC grade methanol (50 µL). The reaction mixture was stirred for 1 hour. The reaction was terminated by addition of aminopropan-2-ol (5 µL). The reaction mixture was stirred for 15 more minutes before precipitation into a mixture acetone:Et₂O (1:1, 60 mL). The copolymer was isolated by filtration, washed extensively with a acetone:Et₂O mixture (2:1) and dried under vacuum. The resulting copolymers were kept protected from light at 4°C.

### Example II

The influence of the chemical structure of hydrophobic side chains on the skin-association of amphiphilic copolymers was assessed by fluorescence microscopy. The surface of full porcine skin was coated with fluorescently-labelled amphiphilic copolymer bearing cholesteryl hydrophobic moieties, pHPMA-chol(2.5%)-fluo. Polymer coated skin was washed three times to remove free or loosely-associated fluorescent polymer prior to analysis. Skin samples were cutted into thin slices (approximately 20µm) using a cryomicrotome and wet-mounted for visualization using a fluorescence microscope. The detailed experimental procedure is described below:
Dermatomed full porcine skin (approximately 2 x 2 cm squares) was washed with phosphate buffered saline and subsequently dried with paper towel before polymer coating. A fluorescently-labelled polymer solution (pHPMA-chol(2.5%)-fluo, 10 µL at 5 mg/mL) in H₂O/EtOH (1:1) was applied on the top layer/stratum corneum of full porcine skin. The polymer solution was evenly distributed/extended with a pipette tip over the skin surface and allowed to dry in a fume cupboard. After 30 min, samples were rinsed extensively with PBS and water and allowed to dry for another 30 min. Samples were frozen and cutted into thin cross section samples of approximately 10 µm using a cryomicrotome. Samples were mounted and observed in a fluorescence microscope equipped FITC filter settings.

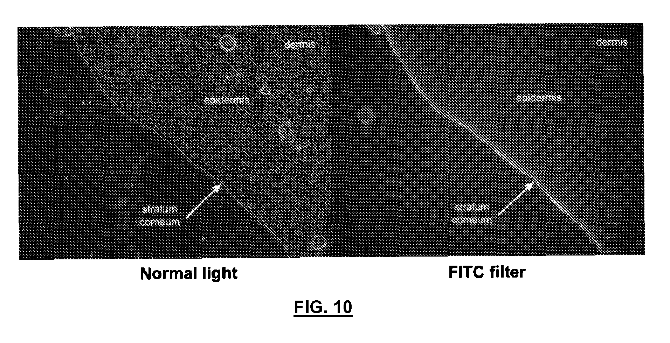

Figure 10 shows a cross section of porcine skin that has been previously coated with fluorescently labelled co-polymer. Microscopy under normal light shows that the treatment does not disturb the normal morphology of skin. When samples were illuminated with light of the appropiate wavelength (FITC filter), localized fluorescence on the top layer (stratum corneum) of the skin was observed. These results prove that the amphiphilic copolymers localize exclusively on the outermost layer of the stratum corneum, forming a thin film without being percutaneously absorbed.

### Example III

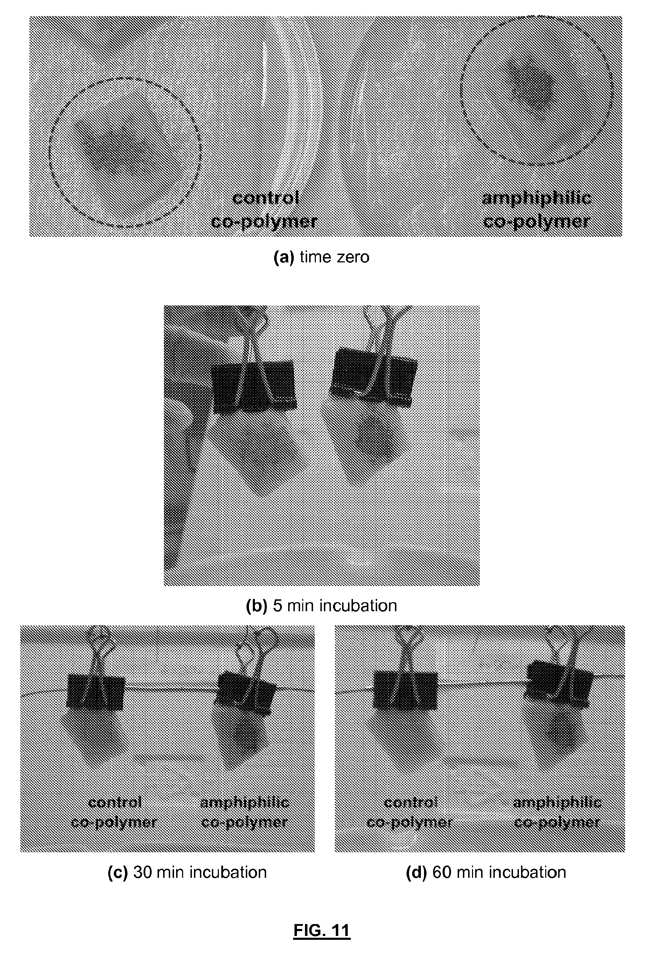

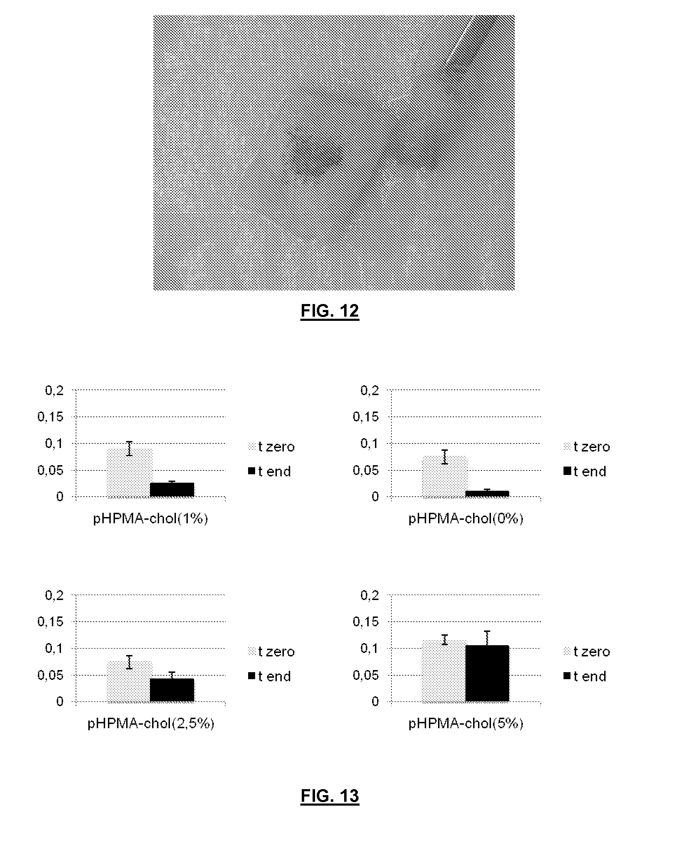

In order to evaluate the resistance of immobilized films against wash off, the stability of the coating films in saline solutions was assessed. In this case, dye-labelled copolymers were employed to allow simple visual monitoring of the desorption process. The surface of full porcine skin was incubated/coated with either dye-labelled amphiphilic copolymers, pHPMA-chol(2.5%)-dye, or control copolymers without pendant hydrophobic groups (pHPMA-dye). Coated skin samples were incubated in a stirred phosphate buffered saline solution and the water resistance of the coating films was visually evaluated at different time points. The detailed experimental procedure is described below:
Dermatomed full porcine skin (aproximately 2 x 2 cm squares) was washed with phosphate buffered saline and subsequently dried with paper towel before polymer coating. Skin was coated with either dye-labelled amphiphilic copolymer (pHPMA-chol(2.5%)-dye, 10 µL at 5 mg/mL in H₂O/EtOH, 1:1) or control copolymer without pendant hydrophobic groups (pHPMA-dye, 10 µL at 5 mg/mL in H₂O/EtOH, 1:1). A dye-labelled polymer solution (amphiphilic copolymer or control) was applied on the top of full porcine skin. The polymer solution was evenly distributed over the skin surface with the help of a pipette tip and allowed to dry in a fume cupboard. Coated skin samples were immersed in a phosphate buffered saline bath (750 mL at 20°C). The saline solution was continously stirred during the course of the experiment using a magnetic bar and a magnetic stirrer (300 rpm). Pictures of the coated skin samples were taken at 0, 5, 30 and 60 minutes incubation (figure 11).
Incubation of skin coated with control copolymers (pHPMA-dye) resulted in rapid desorption of the coating layer during the first minutes. In contrast, films prepared with amphiphilic copolymers (pHPMA-chol(2.5%)-dye) showed high stability against wash off during the course of the experiment. Differences in the wetability behaviour of the copolymers on porcine skin were observed at time zero, depending on the presence or absence of hydrophobic pendant groups. Cholesterol bearing copolymer became more rapidly absorbed, resulting in a more concentric spot. In contrast, spots obtained with control copolymer (without hydrophobic groups) appeared wider, suggesting limited or no polymer-skin interaction.
Films of amphiphilic copolymers on porcine skin can be removed by rubbing the skin surface with a sponge previously soaked with a soapy or alcoholic solution (figure 12).

### Example IV

In order to determine the effect of the concentration of hydrophobic pendant groups on the stability of the coating layer, the amount of copolymer in skin-coated samples was assessed before and after incubation in saline solutions. As shown in the previous example (example 111), dye-labelled copolymers were employed to allow visual monitoring of the desorption process. The surface of full porcine skin was coated with dye-labelled copolymers bearing 0 (control), 1, 2.5 or 5% pendant hydrophobic groups. Coated skin samples were incubated in a saline solution and the water-resistance of the coating films was assessed using a stripping technique followed by spectroscopy quantification. The detailed experimental procedure is described below:
Forearm human skin was washed with ethanol and subsequently dried with paper towel before polymer coating. Skin was coated with either dye-labelled amphiphilic copolymers (pHPMA-chol(1, 2.5 or 5%)-dye, 2 µL at 5 mg/mL in H₂O/EtOH, 1:1) or control copolymer without pendant hydrophobic groups (pHPMA-dye, 2 µL at 5 mg/mL in H₂O/EtOH, 1:1). Six samples (two triplicates) of each dye-labelled copolymer solution (amphiphilic copolymer or control) were applied on the internal side of a human forearm that had been previously shaven. For each sample, the polymer solution was evenly distributed over the skin surface with a pipette tip to cover a circle of approximately 0.5 cm radius and allowed to dry. Before incubation (t_{zero}), 3 samples of each copolymer were stripped using sellotape (5 times per copolymer sample, using 4 x 1.5 cm strips of 3M Scotch Tape). Full forearm was fully immersed in a saline solution (7 L, NaCl at 9 g/L) at room temperature (20°C). After 45 minutes, the forearm was allowed to dry and stripping of the remaining samples was performed (tend).

The material obtained from each stripping (sellotape + stratum corneum/copolymer) was incubated in HPLC grade methanol (3 mL per samples) in order to dissolve the recovered copolymer. After 4 hours, the methanolic solutions were separated from the sellotape strips and centrifuged to eliminate insoluble debris from stratum corneum. The absorption of the resulting solutions at 461 nm was determined using a spectrophotometer and values at t_{zero} and tend were compared for each sample to determine the amount of copolymer desorbed/lost during incubation. Figure 13 shows the loss of coating layers during incubation of skin coated with either amphiphilic (pHPMA-chol(1, 2.5 and 5%)-dye) or control (pHPMA-dye) copolymers.

Results show that there is a direct relationship between concentration of hydrophobic pendant groups and water resistance of the coating layer. Films formed with control copolymers (hydrophilic pHPMA-dye) show poor stability against incubation in saline solutions, resulting in a nearly 90% desorption of the polymer layer. When copolymers bearing low concentration of hydrophobic pendant groups (1 and 2.5%) were used, modest to fair stability against wash off was observed. In contrast, copolymers that contained 5 mol% cholesterol derivatives showed complete stability of the coating films during the course of the experiment. Statistical analysis revealed no significant difference in the amount of coating material after and before incubation. Figure 14 shows the films prepared from pHPMA-chol(5%)-dye before and after incubation. No apparent changes in the shape and the appearance of the deposited spots can be observed.

### Example V

In order to test the performance of these films, copolymer-coated skin samples were evaluated using an experimental procedure designed to assess the water resistance of sunscreen formulations (Agin¹). In sunscreen testing, a product is considered water resistant, if the protection effect remains after 40 minutes of water exposure. Similarly, a product is considered very water resistant, if the protection effect remains after 80 minutes of water exposure. The water resistance test consists of cycles of 20-min immersion intervals, each followed by a 20-min rest/air dry period until the total water exposure time is reached. In the following experiment, the water resistance of films made of amphiphilic copolymers bearing 5 mol% hydrophobic pendant groups (pHPMA-chol(5%)-dye) was evaluated. Similarly as in example IV, the remaining amount of copolymer in polymer-coated skin samples was quantified spectrophotometrically after 20, 40 and 80 min incubation in saline solutions. The detailed experimental procedure is described below:
Inner forearm skin was washed with ethanol and subsequently dried with paper towel before polymer coating. Dye-labelled amphiphilic copolymer (pHPMA-chol(5%)-dye, 2 µL at 5 mg/mL in H₂O/EtOH, 1:1) solution was spotted in triplicate for each time point (t_{zero}, t₂₀ₘᵢₙ t₄₀ₘᵢₙ and t₈₀ₘᵢₙ) on the skin surface of a human forearm. To obtain each sample spot, polymer solution was evenly distributed over the skin surface with a pipette tip to cover a circle of approximately 0.5 cm radius and allowed to dry. At time t_{zero} (before incubation), 3 spotted samples were stripped using sellotape (5 times per copolymer sample, using 4 x 1.5 cm strips of 3M Scotch Tape). Polymer-coated forearm was fully immersed in a saline solution (7 L, NaCl at 9 g/L) at room temperature (20°C) for 20-minute cycles followed by 20-minute rest/air dry period cycles.

Spotted samples were removed by stripping (5 times per copolymer sample, using 4 x 1.5 cm strips of 3M Scotch Tape) after one (20 minutes total incubation, t₂₀ₘᵢₙ), two (40 minutes total incubation, t₄₀ₘᵢₙ) and four (80 minutes total incubation, t₈₀ₘᵢₙ) incubation periods respectively.

The material obtained from each stripping (sellotape + stratum corneum/copolymer) was incubated in a mixture of HPLC grade methanol and 2-propanol (2:1, 5 mL per sample) in order to dissolved the recovered copolymer. After 4 hours, the alcoholic solutions were separated from the sellotape strips and centrifuged to eliminate insoluble debris from stratum corneum. The absorption of the resulting solutions at 461 nm was determined using a spectrophotometer and values at t_{zero}, t₂₀ₘᵢₙ, t₄₀ₘᵢₙ and t₈₀ₘᵢₙ were compared for each sample to determine the amount of copolymer desorbed during incubation. Figure 16 shows the loss of coating layers during incubation of skin coated with amphiphilic pHPMA-chol(5%)-dye copolymers.

Results revealed that films prepared with amphiphilic copolymers bearing 5 mol% cholesterol pendant groups showed excellent water resistance. Coating layers showed complete stability after 4 combined 20-min incubation/20-min air dry cycles. These results prove that this system is able to mantain an active principle immobilized in close contact of the skin, without beeing easily washed off or percutaneously absorbed.

### Example VI

Results obtained in example V, have proved that it is possible to immobilize films containing active principles (in example V, the dye molecules act as an example of an active principles). Cinnamate esters are active ingredients in sunscreen formulations, its primary function is to absorb UV-B radiation from the sun to protect the skin from damage. In order to ensure that the active agent/copolymer conjugation does not affect the activity of the active agent, an amino-derivative of cinnamate ester was conjugated to an amphiphilic copolymer and its ability to absorb UV-B radiation was evaluated by spectrophotometric methods (figure 17). The detailed experimental procedure is described below:

### Copolymer conjugation of 5-aminopentyl-p-methoxycinnamate

pHPMA-chol(2.5%)-TT(10%) (40 mg) and 5-aminopentyl-p-methoxycinnamate (9 mg, 3.4·10⁻⁵ mol) were dissolved in methanol (0.5 mL, HPLC grade) and stirred at room temperature. After 90 min, 1-amino-2-propanol (2 µL) was added to aminolyze any remaining reactive ester and the reaction mixture was further stirred for 20 min. The resulting conjugated copolymer, pHMPA-chol(2.5%)-UV, was isolated by precipitation in a diethyl ether/acetone mixture (1:1), filtered and dried under vacuum until constant weight.

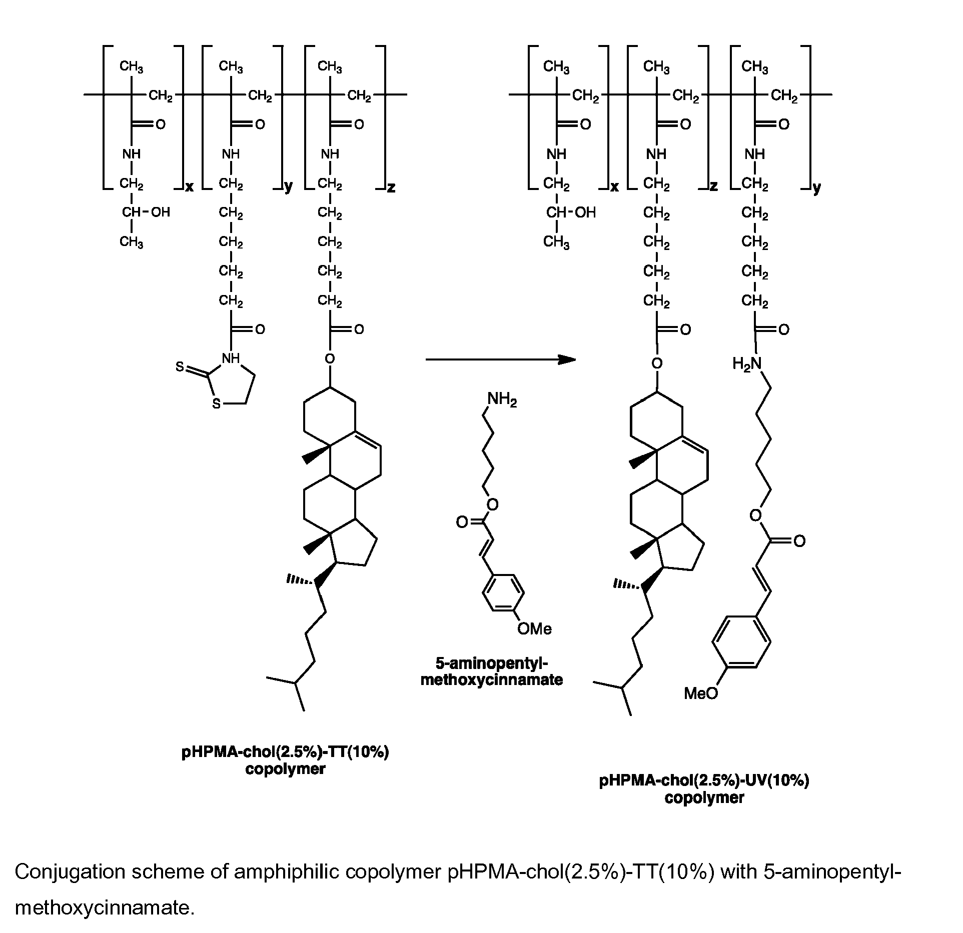

UV/Vis spectra of pHPMA-chol(2.5%), ethyl-p-methoxycinnamate and pHPMA-chol(2.5%)-UV dissolved in methanol were recorded in a spectrophotometer (figure 17). Comparison of recorded spectra shows successful conjugation of cinnamate esters to the amphiphilic copolymer. Polymer conjugation of cinnamate groups does not interfere the absorbance in the target region comprised between 280 and 320 nm.

### Example VII

Another preferred used of this invention includes the controlled release of active principles that had been immobilized on the surface of the skin using the previously described amphiphilic copolymers. In order to test the ability of these copolymers to conjugate active principles and release them through activation of a trigger mechanism such as pH, a model compound was conjugated to an amphiphilic copolymer via a pH sensitive linkage. Release of the active principle from the copolymer conjugate was evaluated *in vitro* using a saline solution (pH 7.4) and a solution that mimicks the acid mantle present on the surface of the skin (acid mantle, pH 4.5 - 5.5). In saline solutions, the neutral pH should not favor release of the active principle, while at a more acidic pH the linkage should not be stable resulting in release of the active principle. The detailed experimental procedure is described below:

### Copolymer conjugation of DMAB via a reversible linkage

To a stirred solution of pHPMA-chol(2.5%)-TT(10%) (50 mg) in methanol (1.0 mL, HPLC grade) was rapidly added hydrazine monohydrate (15 µL, mol). The reaction mixture was stirred at room temperature and after 90 min, DI water was added (10 mL). The resulting solution was dialyzed against DI water for 48 h and freeze-dried to obtain an hydrazine derivate of the amphiphilic copolymer, pHPMA-chol(2.5%)-NH-NH₂.

The hydrazine derivate pHPMA-chol(2.5%)-NH-NH₂ (32 mg) and 4-(dimethylamino)benzaldehyde (DMAB, 18 mg) were dissolved in anhydrous methanol. One drop of glacial acetic acid was added and the reaction mixture was kept in the dark for 48 h. The final conjugate was isolated by precipitation in a diethyl ether/acetone mixture (1:1), filtered and dried under vacuum until constant weight.

pHPMA-chol(2.5%)-DMAB was dissolved in either phosphate buffered saline (pH 7.4) or artificial sweat (pH 5.0) at a final concentration of 1 mg/mL. The resulting solutions were incubated at 33°C and 200 µL aliquots were taken at different time points to quantify the amount of released DMAB. Released DMAB was determined using an adapted method previously described in the literature (Bartos³⁷)_{.}

Amphiphilic copolymer conjugated with DMAB via a pH sensitive linkage showed release of DMAB, when incubated in a solution that mimicked artificial sweat (figure 18). Approximately 45% of the conjugated DMAB was released after 24 h incubation at pH 5. In contrast, incubation of the copolymer conjugate in phosphate buffered saline resulted in low release of DMAB. After 24 h, less than 4% DMAB was released from the copolymer.

### Example VIII - Water Absorption

In order to check the ability of the amphiphilic copolymers to be used as humectants, an *in vitro* test was developed to evaluate the water absortion. In the following assay, the test compounds were prepared, dried in an oven until constant weight, exposed to a 100% humidity chamber for several hours and left to equilibrate at a fixed relative humidity. The amount of water absorbed by the test material was calculated from the weight difference before and after expsoure to humid conditions. In the following experiment, the ability of pHPMA-chol(2.5%)-TT copolymer to absorbe water was quantified. Prior to any experimental, the reactive ester groups were aminolyzed using 1-amino-2-propanol to avoid any possible interferences caused by due to reaction of the polymer with the substrates. The detailed experimental procedure is described below:
Aminolysis of pHPMA-chol(2.5%)-TT with 1-amino-2-propanol

To a stirred solution of pHPMA-chol(2.5%)-TT(10%) (50 mg) in anhydrous methanol (0.5 mL) was added 1-amino-2-propanol (10 µL). The reaction mixture was stirred at room temperature for 90 min to ensure total aminolysis of the reactive groups. The resulting copolymer, pHMPA-chol(2.5%)-AP, was isolated by precipitation in a diethyl ether/acetone mixture (1:1), filtered and dried under vacuum until constant weight.

### Water Absorption

Either a test solution of pHPMA-chol(2.5%)-AP (200 µL at 10 mg/mL in MeOH) or a control solution (200 µL MeOH) was added dropwise on the center of filter paper (10 cm diameter) to form a spot of approximately 2 cm of diameter. This operation was repeated twice for test and control samples in order to have triplicates and perform statistical analysis. The coated filter paper samples were dried in an oven at 140°C under vacuum for 6 hours and the weight of each sample was assessed. Oven-dried samples were incubated overnight in a 100% humidity chamber. On the next morning, samples were removed from the humidity chamber, allowed to equilibrate to a relative humidity of 45% during 6 hours (note: full equilibration was confirmed by constant weight of the samples) and weighted. Water absorption was calculated from the difference of masses between oven-dried samples and samples exposed to humidity (figure 19).

Results showed that filter paper coated with amphiphilic copolymer pHPMA-chol(2.5%)-AP significantly absorbed more water than control filter paper, 0.0068 vs. 0.0112 g of water. The difference in the amount of absorbed water can only be attributed to the effect of the amphiphilic copolymer, which proves the ability of these copolymers to attract and retain water molecules. According to this experiment, the amphiphilic copolymer pHPMA-chol(2.5%)-AP is capable of absorbing approximately 2.2 mg of water per milligram of copolymer.

### BIBLIOGRAPHY

¹ P. P. Agin, Water Resistance and Extended Wear Sunscreens, Dermatol. Clin. 24 (2006) 75-79
² C. H. Alarcon, S. Pennadam and C. Alexander, Stimuli responsive polymers for biomedical applications, Chem. Soc. Rev. 34 (2005) 276-285
³ R. Alvarez-Roman, G. Barreb, R. H. Guy, H. Fessi, Biodegradable polymer nanocapsules containing a sunscreen agent: preparation and photoprotection, European Journal of Pharmaceutics and Biopharmaceutics 52 (2001) 191-195
⁴ J. Bartos, M. Pesez, Colorimetric and Fluorimetric Determination of Aldehydes and Ketones, Pure & Appl. Chem. 51 (1979) 1803-1814
⁵ R. L. Bronaugh, H. I. Maibach, Percutaneous Absorption: Drugs - Cosmetics - Mechanisms - Methodology (Drugs and the Pharmaceutical Sciences). Informa Healthcare; 4th Ed. (2005)
⁶ K. A. Casper, OTC product: band-aid liquid bandage., J. Am. Pharm. Assoc., 46 (2006) 768
⁷ P. Chytil, T. Etrych, C. Konák, M. 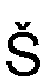 írová, T. Mrkvan, J. Bou 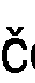 ek, B. 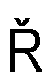 ihová, K. Ulbrich, New HPMA copolymer-based drug carriers with covalently bound hydrophobic substituents for solid tumour targeting, Journal of Controlled Release 127 (2008) 121-130
⁸ W. H. Eaglstein, T. P. Sullivan, P. A. Giordano, B. M. Miskin, A liquid adhesive bandage for the treatment of minor cuts and abrasions, Dermatol. Surg. 28 (2002) 263-7
⁹ P. M. Elias, G. K. Menon, Structural and lipid biochemical correlates of the epidermal permeability barrier, Adv. Lipid Res. 24 (1991) 1-26
¹⁰ B. Ermis, O. Rahmi, T. Ayhan, B. Ozkan, Cushing's syndrome secondary to topical corticosteroids abuse, Clinical Endocrinology 58 (2003) 795-797
¹¹ T. Etrych, M. Jelinkova, B. Rihova, K. Ulbrich, New HPMA copolymers containing doxorubicin bound via pH-sensitive linkage: synthesis and preliminary in vitro and in vivo biological properties, Journal of Controlled Release 73 (2001) 89-102
¹² C. Hayden, M. S. Roberts, H. Benson, Systemic absorption of sunscreen after topical application, The Lancet 350 (1997) 863
¹³ P. L. Honeywell-Nguyen, A. M. de Graaff, H. W. Groenink, J. A. Bouwstra, The in vivo and in vitro interactions of elastic and rigid vesicles with human skin, Biochim. Biophys. Acta 1573 (2002) 130-140
¹⁴ D. K. Jeng, A new, water-resistant, film-forming, 30-second, one-step application iodophor preoperative skin preparation, Am. J. Infect. Control. 29 (2001) 370-6
¹⁵ S. K. Klee, M. Farwick, P. Lersch, Triggered release of sensitive active ingredients upon response to the skin's natural pH, Colloids and Surfaces A: Physicochem. Eng. Aspects 338 (2009) 162-166
¹⁶ M. A. Lampe, A. L. Burlingame, J. Whitney, M. L. Williams, B. E. Brown, E. Roitman, Human stratum corneum lipids: characterization and regional differences, J. Lipid. Res. 24 (1983) 120-30
¹⁷ L. Liu, G. Chen, Marshall, L. Fishman, K. B. Hicks, Pectin Gel Vehicles for Controlled Fragrance Delivery, Drug Delivery 12 (2005) 149-157
¹⁸ A. Misra, R. Pal, S. S. Majumdar, G. P. Talwar, O. Singh, Biphasic testosterone delivery profile observed with two different transdermal formulations, Pharm. Res. 14 (1997) 264-1268
¹⁹ M. Nino, G. Calabrò, P. Santoianni, Topical delivery of active principles: The field of dermatological research, Dermatology Online Journal 16 (2010) 4
²⁰ S. Pattanaargson, N. Hongchinnagorn, P. Hirunsupachot, Y. Sritanaanant, UV Absorption and Photoisomerization of p-Methoxycinnamate Grafted Silicone, Photochemistry and Photobiology 80 (2004) 322-325
²¹ Rachel Petkewich, Liquid Bandages, Chemical Engineering News 86 (2008) 61
²² J. Saint Laumer, E. Frerot, A. Herrmann, Controlled Release of Perfumery Alcohols by Neighboring-Group Participation. Comparison of the Rate Constants for the Alkaline Hydrolysis of 2-Acyl-, 2-(Hydroxymethyl)-, and 2-Carbamoylbenzoates, Helvetica Chimica Acta 86 (2003) 2871
²³ C. Santos-Maia, W. Mehnert, M. Schaller, H. C. Korting, A. Gysler, A. Haberland, M. Schaefer-Korting, Drug targeting by solid lipid nanoparticles for dermal use, J. Drug Target. 10 (2002) 489-495
²⁴ F. Rippke, V. Schreiner, H. J. Schwanitz, The acidic milieu of the horny layer: new findings on the physiology and pathophysiology of skin pH, Am. J. Clin. Dermatol. 3 (2002) 261-72
²⁵ M. H. Schmid-Wendtner, H. C. Korting, The pH of the skin surface and its impact on the barrier function, Skin Pharmacol. Physiol. 19 (2006) 296-302
²⁶ A. J. Singer, H. C. Thode, A review of the literature on octylcyanoacrylate tissue adhesive, Am. J. Surg. 187 (2004) 238-48
²⁷ B. Sondell, L. E. Thornhell, T. Stigbrand, T. Egelrud, lmmunolocalization of Stratum Corneum Chymotryptic Enzyme in Human Skin and Oral Epithelium with Monoclonal Antibodies: Evidence of a Proteinase Specifically Expressed in Keratinizing Squamous Epithelial, The Journal of Histochemistry and Cytochemistry 42 (1994) 459-465
²⁸ C. Surber, A. F. Davis, Bioavailability and Bioequivalence of Dermatological Formulations, Dermatological and Transdermal Formulations 119 (2002) 401-498
²⁹ A. Thielitz, H. Gollnick, Topical retinoids in acne vulgaris: update on efficacy and safety, Am. J. Clin. Dermatol. 9 (2008) 369-81
³⁰ K. Ulbrich, V. Subr, J. Strohalm, D. Plocová, M. Jelínková and B. Rihová, Polymeric drugs based on conjugates of synthetic and natural macromolecules. I. Synthesis and physico-chemical characterisation, J Control Release 64 (2000) 63-79
³¹ K. Ulbrich, V. Subr, Polymeric anticancer drugs with pH-controlled activation, Adv. Drug Deliv. Rev. 56 (2004) 1023-50
³² C. V. Wa, H. I. Maibach, Mapping the human face: biophysical properties, Skin Research and Technology 16 (2010) 38-54
³³ S. Q. Wang, Y. Balagula, U. Osterwalder, Photoprotection: a Review of the Current and Future Technologies, Dermatologic Therapy 23 (2010) 31-47
³⁴ M. R. Prausnitz, R. Langer, Transdermal drug delivery, Nat. Biotechnol. 26 (2008) 1261-1268
³⁵ C. H. Alarcon, S. Pennadam, C. Alexander, Stimuli responsive polymers for biomedical applications, Chem. Soc. Rev. 34 (2005) 276-285
³⁶ V. Subr, K. Ulbrich, Synthesis and properties of new N-(2-hydroxypropyl)-methacrylamide copolymers containing thiazolidine-2-thione reactive groups, Reactive & Functional Polymers 66 (2006) 1525-1538
³⁷ J. Martos, M. Pesez, Colorimetric and fluorimetric determination of aldehydes and ketones, Pure & Appl. Chem. 51 (1971) 1803-1814

## Claims

1. A water-soluble polymer-active ingredient conjugate comprising:
- at least one hydrophilic copolymer chain comprising monomers derived from acrylic acid, methacrylic acid or any water-soluble vinyl monomer in any combination thereof; and
- at least one active ingredient linked to the copolymer chain through a linker group having at least one covalent bond;
**characterized in that** the copolymer chain has a number-average molecular weight of at least 50,000 g/mol and comprises at least 1 mol% of monomers having at least one pendant hydrophobic group able to bind to the skin of an animal by hydrophobic interactions.

2. The water-soluble conjugate of claim 1, wherein each pendant hydrophobic group is a cholesterol moiety.

3. The water-soluble conjugate of claims 1 or 2, wherein the hydrophilic copolymer chain is poly[N-(2-hydroxypropyl)methacrylamide].

4. The water-soluble conjugate of any one of claims 1 to 3, wherein the copolymer chain comprises at least 3 mol% of monomers having at least one pendant hydrophobic group.

5. The water-soluble conjugate of any one of claims 1 to 4, wherein the copolymer chain comprises at least 5 mol% of monomers having at least one pendant hydrophobic group.

6. The water-soluble conjugate of any one of claims 1 to 5, wherein the copolymer chain has a number-average molecular weight of at least 70,000 g/mol.

7. The water-soluble conjugate of any one of claims 1 to 6, wherein the copolymer chain has a number-average molecular weight of at least 100,000 g/mol.

8. The water-soluble conjugate of any one of previous claims 1 to 7, wherein the active ingredient is selected from humectants, sunscreens, fragrances, disinfectants, insect repellents, pigments, colourings or any other cosmetically or dermatologically active ingredient or drug.

9. The water-soluble conjugate of any one of claims 1 to 8, wherein the linker group bonding the active ingredient to the copolymer chain is non-cleavable.

10. The water-soluble conjugate of claim 9, wherein the non-cleavable linker group is selected from the group consisting of hydroxyls, carboxyls, thiols, amines, esters, ethers, amides, anhydrides, acetals, hydroxamates, carbonates, carbamates and ureas.

11. The water-soluble conjugate of any one of claims 1 to 8, wherein the linker group bonding the active ingredient to the copolymer is cleavable through an exogenous trigger mechanism.

12. The water-soluble conjugate of claim 11, wherein the trigger mechanism is selected from low pH, enzymes, reducing media or light.

13. The water-soluble conjugate of claims 11 or 12, wherein the cleavable linker group is selected from the group consisting of hydrazones, acetals, ketals, mixed acetal-ketals, orthoesters, imines, amides, amide (peptides) or esters.

14. The water-soluble conjugate of any one of the previous claims further comprising a pharmaceutically or cosmetically acceptable solvent.

15. Method to deliver an active ingredient over the skin of an animal, comprising the steps of applying the water-soluble conjugate of any one of previous claims 1-14 over the skin of the animal and allowing the solvent to evaporate, thereby forming a film over the skin having the active ingredient covalently attached thereto.

16. Method to deliver an active ingredient over the skin of an animal according to claim 15 further comprising the step of applying to the film an exogenous trigger mechanism, which cleaves the cleavable linker group bonding the active ingredient to the copolymer, thereby allowing the controlled release of the active ingredient.

17. Use of any one of the polymer-active ingredient conjugates of previous claims 1-14 to deliver an active ingredient over the skin of an animal.

18. Use according to claim 17 wherein the active ingredient is released by applying to the film resulting from the application to the skin of any one of the conjugates of previous claims 1-14 an exogenous trigger mechanism, which cleaves the cleavable linker group bonding the active ingredient to the copolymer chain.
